(19) Europäisches Patentamt
European Patent Office
Office européen des brevets

(11) EP 4 458 344 A1

(12) **EUROPEAN PATENT APPLICATION**
published in accordance with Art. 153(4) EPC

(43) Date of publication:
**06.11.2024 Bulletin 2024/45**

(21) Application number: **22916017.1**

(22) Date of filing: **26.12.2022**

(51) International Patent Classification (IPC):
***A61K 6/20*** *(2020.01)* ***A61K 6/17*** *(2020.01)*
***A61K 6/60*** *(2020.01)* ***A61K 6/62*** *(2020.01)*
***A61K 6/65*** *(2020.01)* ***A61K 6/76*** *(2020.01)*
***A61K 6/78*** *(2020.01)* ***A61K 6/802*** *(2020.01)*
***A61K 6/818*** *(2020.01)*

(52) Cooperative Patent Classification (CPC):
**A61K 6/17; A61K 6/20; A61K 6/60; A61K 6/62; A61K 6/65; A61K 6/76; A61K 6/78; A61K 6/802; A61K 6/818**

(86) International application number:
**PCT/JP2022/047911**

(87) International publication number:
**WO 2023/127794 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2021 JP 2021213432**

(71) Applicant: **Kuraray Noritake Dental Inc.**
**Kurashiki-shi, Okayama 710-0801 (JP)**

(72) Inventors:
- **KASHIKI Nobusuke**
  **Miyoshi-shi, Aichi 470-0293 (JP)**
- **SAKAMOTO Hiroyuki**
  **Miyoshi-shi, Aichi 470-0293 (JP)**

(74) Representative: **D Young & Co LLP**
**3 Noble Street**
**London EC2V 7BQ (GB)**

(54) **DENTAL CURABLE COMPOSITION AND DENTAL PROSTHESIS COMPRISING BASE MATERIAL AND RESIN LAYER**

(57) The present invention provides a dental curable composition and a dental prosthesis therefrom, capable of imparting fluorescence to the base material, as well as exhibiting outstanding operability, reducing shade changes in the base material, and providing excellent abrasion resistance, gloss retention, and fluorescence durability in the cured product. The present invention relates to a dental curable composition comprising a polymerizable compound (A), a polymerization accelerator (B), a fluorescent agent (C), and a polymerization initiator (D), wherein the polymerization accelerator (B) comprises a polyfunctional thiol (B-1) having two or more mercapto groups per molecule.

EP 4 458 344 A1

## Description

TECHNICAL FIELD

The present invention relates to dental curable compositions and dental prostheses. More specifically, the invention relates to a dental curable composition for coating base materials, and to a dental prosthesis comprising a base material and a resin layer.

BACKGROUND ART

In the past, metals were commonly used for dental prostheses (for example, such as veneer crowns, dental caps, crowns, and post crowns).

However, metals have the drawback of lacking aesthetics, and occasionally causing allergic reactions due to leaching of metal. To address the issues associated with the use of metals, ceramic materials such as aluminum oxide (alumina), zirconium oxide (zirconia), fused quartz, and lithium silicate glass, as well as acrylic resins, and composite materials containing polymer resins and inorganic fillers have been used for dental products as alternatives to metal. Zirconia, in particular, offers superior aesthetics and strength, and has seen a rise in demand, especially due to its increasing affordability in recent years.

To achieve improved aesthetics in the oral cavity, it is necessary for dental prostheses to mimic the look of natural teeth. However, achieving a natural tooth-like appearance, especially in terms of transparency, gloss (glaze or shine), and shade, is difficult with zirconia (a sintered body) alone. This issue is addressed by a veneer crown aiming to replicate the appearance of natural teeth by baking a ceramic material called porcelain onto the exposed surface of a zirconia frame (base material), rather than leaving the zirconia exposed. Such dental products are called porcelain-fused-to-zirconia (PFZ) crowns.

In another technique that can achieve an aesthetic appearance similar to natural teeth, an adhesive is applied to a high-strength ceramic frame used as a base material, and a polymerizable composition, primarily composed of a polymerizable monomer, an inorganic filler, and a polymerization catalyst (commonly known as a dental composite resin) is built up in layers and allowed to polymerize and cure.

Patent Literatures 1 to 5 represent known examples of such related art.

Patent Literature 1 discloses a dental prosthesis comprising a resin layer using a polymerizable monomer, and a base material, wherein the base material comprises zirconia, and is coated with the resin layer.

Patent Literature 2 discloses a dental prosthesis in which a polymerizable composition (dental composite resin), built up on a ceramic frame (base material), is polymerized and cured.

Patent Literature 3 discloses coating the surface of a dental material with a coating material that comprises a (meth)acrylate monomer containing inorganic fine particles that have been surface-modified with alkoxysilane having unsaturated double bonds.

Patent Literature 4 reports an aesthetic dental restoration or a method for protecting the tooth structure, whereby a coating material composition comprising an $\alpha$-diketone compound, an amine compound, and a polymerizable monomer is polymerized and cured after it is coated on the surface of a dental restoration.

Patent Literature 5 discloses a dental curable composition comprising a polymerizable monomer, an inorganic filler, a polyorganosilsesquioxane particle, and a polymerization initiator, demonstrating excellence in gloss polishability.

Human natural teeth exhibit fluorescence. In cases where a dental prosthesis lacks fluorescence, only the prosthesis portion of the teeth will not emit fluorescence in an environment under ultraviolet light exposure, for example, such as in an amusement facility with blacklight, giving the impression that the tooth is absent.

To address these issues and improve oral aesthetics, it is feasible to impart fluorescence to dental prostheses by incorporating a fluorescent agent in dental prostheses. Zirconia sintered bodies containing a fluorescent agent are known, as described in Patent Literature 6, for example.

Dental compositions containing a polythiol compound are also known, as disclosed in Patent Literatures 7 and 8, for example.

CITATION LIST

Patent Literature

Patent Literature 1: JP 2018-89312 A

Patent Literature 2: JP 2001-149385 A

Patent Literature 3: JP 2005-154312 A
Patent Literature 4: JP 2021-54813 A
Patent Literature 5: JP 2016-153382 A
Patent Literature 6: WO2019/026809
Patent Literature 7: JP 2020-509060 T
Patent Literature 8: JP 2019-116471 A

SUMMARY OF INVENTION

Technical Problem

Typically, PFZ is created by applying a slurry containing ceramic materials, which transform into porcelain, onto a base material, followed by firing at temperatures of several hundred degrees Celsius to fuse the porcelain to the base material. To prevent defects during firing, it is therefore required to choose a porcelain material with a coefficient of thermal expansion similar to that of the base material. For example, when a zirconia sintered body is used as the base material, it is essential to choose a porcelain ceramic material with a coefficient of thermal expansion close to that of the zirconia sintered body.

Additionally, in the process of PFZ fabrication, it has been required to heat the porcelain to a temperature at which it melts (typically ranging from about 900 to 1,000°C) using an electric furnace, after applying a porcelain slurry to the zirconia surface. There accordingly is a need for a more convenient method to produce aesthetically pleasing zirconia prostheses without using porcelain.

Patent Literature 1 discloses a method for enhancing the translucency of base material zirconia with a prosthesis obtained by coating a polymerizable monomer. Patent Literature 2 discloses a dental prosthesis produced by building up a polymerizable composition on an alumina base material.

However, with the polymerizable monomers disclosed, both techniques face a challenge in maintaining sufficient durability to sustain gloss in the demanding environment of the oral cavity.

Another issue is that Patent Literature 1 involves a possible shift in the shade of the resin layer coating the base material due to polymerization and cure, though the method provides satisfactory translucency. That is, there is room for improvement in reducing shade changes in coating the base material, in addition to providing transparency.

The photopolymerizable dental coating material composition described in Patent Literature 3 involves an issue of undergoing gelation during storage, resulting in decline in surface curability over time. The photopolymerizable dental coating material composition described in Patent Literature 4 faces a challenge in maintaining sufficient durability to sustain gloss in the demanding environment of the oral cavity, and no consideration is given concerning ceramic materials.

The dental curable composition described in Patent Literature 5 excels in properties such as transparency. However, there is still room for improvement in glossiness to achieve an appearance comparable to natural teeth, as well as in the operability in building up the dental curable composition.

The fluorescent agent-containing zirconia sintered body described in Patent Literature 6 may undergo shade changes due to the influence of the fluorescent agent, and, in order to achieve an appearance comparable to natural teeth, further improvements are needed in reducing shade changes in the base material.

Patent Literature 7 relates to flowable composites. The dental composition of Patent Literature 7 exhibits excessive viscosity as a composition, and there is a need for improvement in terms of operability when used for coating of dental prostheses such as zirconia. Additionally, Patent Literature 7 does not address abrasion resistance, gloss retention, and fluorescence durability.

The dental resin composition describes in Patent Literature 8 is intended for use as a solid block of resin for dental milling purposes. Consequently, the dental resin composition described in Patent Literature 8 is not meant for coating of dental prostheses such as zirconia, which involves application of a dental resin composition to a base material. That is, the specific challenges related to coating applications for dental prostheses are not taken into account. These include considerations such as the operability to form a uniform film, reduction of shade changes in the base material, the provision of fluorescence to the base material upon curing the composition built up on the base material, and the gloss retention and fluorescence durability of the cured product.

It is an object of the present invention to provide a dental curable composition and a dental prosthesis therefrom, capable of imparting fluorescence to the base material, as well as exhibiting outstanding operability, reducing shade changes in the base material, and providing excellent abrasion resistance, gloss retention, and fluorescence durability in the cured product.

Solution to Problem

The present inventors conducted intensive studies to find a solution to the foregoing issues, and found that a

dental curable composition comprising a polyfunctional thiol (B-1) having two or more mercapto groups per molecule, and a fluorescent agent (C) along with other components adheres to the base material both chemically and mechanically, and allows the resulting coating layer to maintain adequate hardness, glossiness, and light reflection, providing a way to conveniently retain natural gloss and fluorescence in the dental prosthesis over an extended time period. This led to the completion of the present invention after further examinations.

Additionally, a dental prosthesis comprising a base material and a resin layer was found to be useful when the resin layer comprises a cured product of polymerization of a curable composition comprising a polymerizable compound (A), a polyfunctional thiol (B-1), a fluorescent agent (C), and a polymerization initiator (D), and is coated over the base material with a certain thickness. Building upon this finding, the present inventors conducted additional examinations, ultimately resulting in the completion of the present invention.

Specifically, the present invention relates to the following.

[1] A dental curable composition comprising a polymerizable compound (A), a polymerization accelerator (B), a fluorescent agent (C), and a polymerization initiator (D),
wherein the polymerization accelerator (B) comprises a polyfunctional thiol (B-1) having two or more mercapto groups per molecule.

[2] The dental curable composition according to [1], wherein the polyfunctional thiol (B-1) comprises a polyfunctional thiol having 2 to 4 mercapto groups per molecule.

[3] The dental curable composition according to [1] or [2], wherein the mercapto groups in the polyfunctional thiol (B-1) are bound to a secondary carbon atom or a tertiary carbon atom.

[4] The dental curable composition according to any one of [1] to [3], wherein the fluorescent agent (C) comprises an organic fluorescent agent (C-2).

[5] The dental curable composition according to [4], wherein the content of the organic fluorescent agent (C-2) is 0.001 to 10 mass%.

[6] The dental curable composition according to any one of [1] to [5], wherein the mass ratio (B-1):(C) of the content of the polyfunctional thiol (B-1) and the content of the fluorescent agent (C) is 100:0.001 to 100:600.

[7] The dental curable composition according to any one of [1] to [6], wherein the polymerizable compound (A) comprises a urethane (meth)acrylate oligomer (A-1) and/or a polymerizable monomer (A-2).

[8] The dental curable composition according to [7], wherein the polymerizable monomer (A-2) is a (meth)acrylic polymerizable monomer.

[9] The dental curable composition according to any one of [1] to [8], wherein the polymerization initiator (D) comprises a photopolymerization initiator (D-1).

[10] The dental curable composition according to any one of [1] to [9], which further comprises a filler (E).

[11] The dental curable composition according to [10], wherein the filler (E) comprises a hydrophobic silica (E-1).

[12] The dental curable composition according to [11], wherein the hydrophobic silica (E-1) has an average primary particle diameter of 15 to 100 nm.

[13] The dental curable composition according to any one of [10] to [12], wherein the content of the filler (E) is 0.1 to 20 mass%.

[14] The dental curable composition according to any one of [1] to [13], which further comprises a polysiloxane (F).

[15] The dental curable composition according to [14], wherein the polysiloxane (F) is liquid at 25°C.

[16] The dental curable composition according to [14] or [15], wherein the polysiloxane (F) comprises a polysilsesquioxane (F-1).

[17] The dental curable composition according to any one of [1] to [16], wherein the dental curable composition is a coating agent for coating a base material.

[18] A dental prosthesis comprising a resin layer and a base material, the resin layer being a layer cured through polymerization of a dental curable composition of any one of [1] to [17].

[19] The dental prosthesis according to [18], wherein the base material comprises at least one selected from the group consisting of zirconia, alumina, fused quartz, lithium silicate glass, an acrylic resin, and a composite material containing a polymer resin and an inorganic filler.

[20] The dental prosthesis according to [19], wherein the zirconia comprises zirconia, and a stabilizing agent capable of preventing a phase transformation of zirconia.

[21] The dental prosthesis according to any one of [18] to [20], wherein the resin layer has a thickness of 1 μm to 500 μm.

Advantageous Effects of Invention

The present invention has enabled the provision of a dental curable composition and a dental prosthesis therefrom, capable of imparting fluorescence to the base material, as well as exhibiting outstanding operability, reducing

shade changes in the base material, and providing excellent abrasion resistance, gloss retention, and fluorescence durability in the cured product.

A dental curable composition according to the present invention exhibits excellent curability, allowing for polymerization and curing in a short time period.

The present invention has also enabled the provision of a dental curable composition and a dental prosthesis therefrom, whereby the surface-coating resin layer exhibits adequate hardness, and chemically and mechanically adheres to the base material to improve abrasion resistance, allowing for protection of the opposing natural teeth or the prosthesis or base material itself against wear, and retaining fluorescence and gloss over extended time periods.

## DESCRIPTION OF EMBODIMENTS

The following describes each component of a dental curable composition of the present invention in detail. It should be noted that, in this specification, "ordinary temperature" means 25°C, strictly speaking.

### <Polymerizable Compound (A)>

The polymerizable compound (A) included in the present invention is described first.

The polymerizable compound (A) included in the present invention preferably comprises a urethane (meth)acrylate oligomer (A-1). The urethane (meth)acrylate oligomer (A-1) is used to impart hardness, abrasion resistance, and toughness to a cured product of a dental curable composition of the present invention.

The urethane (meth)acrylate oligomer (A-1) also contributes to the curability and toughness of the cured product when combined with the polymerizable monomer (A-2) described below.

In this specification, "(meth)acryl" means both methacryl and acryl, and "(meth)acryloyl" means both methacryloyl and acryloyl.

The urethane (meth)acrylate oligomer (A-1) used in a dental curable composition of the present invention is an oligomer having a urethane bond (-NHC(O)O-), and may have at least one structure selected from the group consisting of a polyester, a polyether, a polycarbonate, a polyurethane, and a poly-conjugated diene per molecule, in addition to the urethane bond.

In view of the curability, viscosity, and coatability of the composition, the urethane (meth)acrylate oligomer (A-1) has a viscosity at 25°C of preferably 4,000 to 250,000 mPa·s, more preferably 6,000 to 200,000 mPa·s, even more preferably 8,000 to 150,000 mPas.

The viscosity can be measured with, for example, a B-type viscometer (Brookfield viscometer) under 25°C, 20 rpm conditions.

In view of the hardness of the cured product, a cured film (cured product) of the urethane (meth)acrylate oligomer (A-1) has a pencil hardness of preferably F or higher, more preferably H or higher, even more preferably 2H or higher.

The pencil hardness of the cured film can be measured following, for example, JIS K 5600-5-4:1999.

When combined with the polymerizable monomer (A-2) described below, the urethane (meth)acrylate oligomer (A-1) can impart desirable curability and viscosity, while providing desirable hardness to the cured product.

Examples of the urethane (meth)acrylate oligomer (A-1) are provided below, including commercially available products. In view of enhancing the effectiveness of the present invention, a certain preferred embodiment is, for example, a dental curable composition in which the urethane (meth)acrylate oligomer (A-1) is a urethane acrylate oligomer.

Examples of the urethane (meth)acrylate oligomer (A-1) include:

Urethane acrylate oligomers EBECRYL 8807, EBECRYL 8465, EBECRYL 8800, EBECRYL 4101, EBECRYL 4201, and KRM 7735 commercially available from DAICEL-ALLNEX LTD.;
Urethane acrylate oligomers UV1700B, UV-6300B, UV7550B, UV-7600B, UV-7605B, UV-7630B, UV-7640B, and UV-7650B commercially available from Mitsubishi Chemical Corporation;
Urethane acrylate oligomer CN9006NS commercially available from Sartomer Company, Exton, PA;
Pentaerythritol triacrylate hexamethylene diisocyanate urethane prepolymer UA-306H, pentaerythritol triacrylate toluene diisocyanate urethane prepolymer UA-306T, pentaerythritol triacrylate isophorone diisocyanate urethane prepolymer UA-306I, and dipentaerythritol pentaacrylate hexamethylene diisocyanate urethane prepolymer UA-510H commercially available from Kyoeisha Chemical Co., Ltd.; and
Urethane acrylate oligomers UN-2600, UN-6202, and UN-6304 (urethane acrylate oligomers with polyether skeletons) commercially available from Negami Chemical Industrial Co., Ltd.

Preferred among these examples are urethane acrylate oligomer UV1700B (viscosity at 25°C: 40,000 to 100,000 mPa·s; pencil hardness of cured film: 4H), pentaerythritol triacrylate hexamethylene diisocyanate urethane prepolymer UA-306H (viscosity at 25°C: 10,000 to 40,000 mPa·s; pencil hardness of cured film: 6H), and pentaerythritol triacrylate

toluene diisocyanate urethane prepolymer UA-306T (viscosity at 25°C: 10,000 to 40,000 mPa·s; pencil hardness of cured film: 5H). These may be used alone, or two or more thereof may be used in combination. It is also possible to use a polymer of mixtures of these.

In view of curability, hardness, viscosity, and coatability, the content of urethane (meth)acrylate oligomer (A-1) is preferably 25 to 55 mass%, more preferably 30 to 50 mass%, even more preferably 35 to 45 mass% in a total amount of the dental curable composition.

The content of urethane (meth)acrylate oligomer (A-1) is preferably 30 to 80 mass%, more preferably 35 to 75 mass%, even more preferably 40 to 70 mass% in total 100 mass% of polymerizable compound (A).

The polymerizable compound (A) included in the present invention preferably comprises a polymerizable monomer (A-2). In the present invention, the polymerizable monomer (A-2) is used to enhance curability, and adhesive properties to the base material.

The polymerizable monomer (A-2) also contributes to the curability of the cured product when combined with the urethane (meth)acrylate oligomer (A-1) described above.

The polymerizable monomer (A-2) may be any known polymerizable monomer used for dental compositions.

The polymerizable monomer (A-2) may be a monomer having no acidic group.

Specific examples of the polymerizable monomer (A-2) include esters of acids such as α-cyanoacrylic acid, (meth)acrylic acid, α-halogenated acrylic acid, crotonic acid, cinnamic acid, sorbic acid, maleic acid, and itaconic acid; and (meth)acrylamides, (meth)acrylamide derivatives, vinyl esters, vinyl ethers, mono-N-vinyl derivatives, and styrene derivatives. Particularly preferred for use are (meth)acrylic polymerizable monomers representing (meth)acrylic acid esters or (meth)acrylamides.

Examples of the (meth)acrylic polymerizable monomers as (meth)acrylic acid ester polymerizable monomers or (meth)acrylamide polymerizable monomers include monofunctional monomers such as monofunctional (meth)acrylates, and monofunctional (meth)acrylamides, and polyfunctional monomers such as bifunctional (meth)acrylates, and tri- and higher functional (meth)acrylates.

In relation to polymerizable monomer (A-2), "polyfunctional monomer" means a monomer having two or more polymerizable groups, such as vinyl groups, (meth)acryloyl groups, and (meth)acrylamide groups, excluding the mercapto groups of polyfunctional thiol (B-1).

Preferred examples of the polymerizable monomer (A-2) are as follows.

· Monofunctional (meth)acrylate monomers and monofunctional (meth)acrylamide monomers (a-1):

aliphatic monofunctional (meth)acrylate monomers such as methyl (meth)acrylate, ethyl (meth)acrylate, n-propyl (meth)acrylate, n-butyl (meth)acrylate, isobutyl (meth)acrylate, 2-ethylhexyl (meth)acrylate, lauryl (meth)acrylate, tridecyl (meth)acrylate, stearyl (meth)acrylate, cyclohexyl (meth)acrylate, 2-(N,N-dimethylamino)ethyl (meth)acrylate, 2,3-dibromopropyl (meth)acrylate, 2-hydroxyethyl (meth)acrylate, 2-hydroxypropyl (meth)acrylate, 6-hydroxyhexyl (meth)acrylate, 10-hydroxydecyl (meth)acrylate, glycidyl (meth)acrylate, tetrahydrofurfuryl (meth)acrylate, allyl (meth)acrylate, 2-ethoxyethyl (meth)acrylate, methoxydiethylene glycol (meth)acrylate, methoxytetraethylene glycol (meth)acrylate, methoxypolyethylene glycol (meth)acrylate, phenoxyhexaethylene glycol (meth)acrylate, dicyclopentenyl (meth)acrylate, isobornyl (meth)acrylate, propylene glycol mono(meth)acrylate, glycerin mono(meth)acrylate, erythritol mono(meth)acrylate, pentaerythritol mono(meth)acrylate, dipentaerythritol mono(meth)acrylate, caprolactone-modified tetrahydrofurfuryl (meth)acrylate, caprolactone-modified dipentaerythritol (meth)acrylate, and caprolactone-modified 2-hydroxyethyl (meth)acrylate;
aromatic monofunctional (meth)acrylate monomers such as benzyl (meth)acrylate, phenyl (meth)acrylate, and phenoxydiethylene glycol (meth)acrylate;
monofunctional (meth)acrylamide monomers such as 4-(meth)acryloylmorpholine, N-methylol(meth)acrylamide, N-hydroxyethyl (meth)acrylamide, and N,N-bis(2-hydroxyethyl) (meth)acrylamide; and
halogen-containing monofunctional (meth)acrylate monomers such as (meth)acryloyloxydodecylpyridinium bromide, (meth)acryloyloxydodecylpyridinium chloride, (meth)acryloyloxyhexadecylpyridinium chloride, and (meth)acryloyloxydecylammonium chloride.

The symbol n means "normal."

· Bifunctional (Meth)Acrylate Monomers (a-2):

aliphatic bifunctional (meth)acrylate monomers such as ethylene glycol di(meth)acrylate, diethylene glycol di(meth)acrylate, triethylene glycol di(meth)acrylate, tetraethylene glycol di(meth)acrylate, polyethylene glycol di(meth)acrylate, 1,3-butylene glycol di(meth)acrylate, propylene glycol di(meth)acrylate, tripropylene glycol di(meth)acrylate, polypropylene glycol di(meth)acrylate, neopentyl glycol di(meth)acrylate, propoxylated (2)neopentyl glycol di(meth)acrylate, glycerin di(meth)acrylate, 1,6-hexanediol di(meth)acrylate, 1,9-nonanediol di(meth)acrylate, 1,10-decanediol di(meth)acrylate, 2-hydroxy-3-methacrylpropyl (meth)acrylate, 1,2-bis[3-(meth)acryloyloxy-2-hydroxypropoxy]ethane, pentaerythritol di(meth)acrylate, [2,2,4-trimethylhexamethylenebis(2-carbamoyloxyethyl)]di(meth)acrylate, 7,7,9-trimethyl-4,13-dioxa-3,14-dioxo-5,12-diazahexadecane-1,16-diol di(meth)acrylate, neopentyl glycol hydroxypivalic acid ester di(meth)acrylate, trimethylolethane di(meth)acrylate, and trimethylolpropane di(meth)acrylate;

aromatic bifunctional (meth)acrylate monomers such as bisphenol A diglycidyl acrylate (2,2-bis[4-[3-acryloyloxy-2-hydroxypropoxy]phenyl]propane), bisphenol A diglycidyl methacrylate (2,2-bis[4-[2-hydroxy-3-methacryloyloxypropoxy]phenyl]propane, commonly known as Bis-GMA), 2,2-bis[4-(meth)acryloyloxyethoxyphenyl]propane, 2,2-bis[4-acryloyloxypolyethoxyphenyl]propane, 2,2-bis[4-methacryloyloxypolyethoxyphenyl]propane (with 2.6 moles of ethylene added), and caprolactone-modified hydroxypivalic acid neopentyl glycol ester di(meth)acrylate; and

alicyclic bifunctional (meth)acrylate monomers such as tricyclodecanedimethanol di(meth)acrylate.

· Tri- and Higher-Functional (Meth)Acrylate Monomers (a-3):

trifunctional (meth)acrylate monomers such as trimethylolpropane tri(meth)acrylate, trimethylolethane tri(meth)acrylate, trimethylolmethane tri(meth)acrylate, tetramethylolmethane tri(meth)acrylate, ethoxylated trimethylolpropane tri(meth)acrylate, propoxylated trimethylolpropane tri(meth)acrylate, glycerin tri(meth)acrylate, ethoxylated glycerin tri(meth)acrylate, tris-(2-(meth)acryloyloxyethyl)isocyanurate, ethoxylated (3)trimethylolpropane tri(meth)acrylate, pentaerythritol tri(meth)acrylate, and caprolactone-modified tris-(2-acryloyloxyethyl)isocyanurate;

tetrafunctional (meth)acrylate monomers such as dimethylolpropane tetra(meth)acrylate, pentaerythritol tetra(meth)acrylate, dipentaerythritol tetra(meth)acrylate, ethoxylated pentaerythritol tetra(meth)acrylate, ethoxylated dipentaerythritol tetra(meth)acrylate, and N,N-(2,2,4-trimethylhexamethylene)bis[2-(aminocarboxy)propane-1,3-diol]tetra(meth)acr ylate; and

penta- and higher-functional (meth)acrylate monomers such as dipentaerythritol hexa(meth)acrylate, dipentaerythritol penta(meth)acrylate, and 2,2,6,6-tetrakis(acryloyloxymethyl)-4-oxaheptane-1 ,7-diol 1-(meth)acrylate.

In view of curability, preferred as the polymerizable monomer (A-2) are tri- and higher functional (meth)acrylate monomers (a-3). In view of ease of handling and safety, more preferred are pentaerythritol tri(meth)acrylate, pentaerythritol tetra(meth)acrylate, dipentaerythritol hexa(meth)acrylate, dipentaerythritol penta(meth)acrylate, and ethoxylated (3)trimethylolpropane triacrylate.

The polymerizable monomer (A-2) may be incorporated alone, or two or more thereof may be incorporated in combination. For example, the polyfunctional monomers (preferably, polyfunctional (meth)acrylate monomers) may be incorporated alone, or two or more thereof may be incorporated in combination.

In view of curability, hardness, viscosity, and coatability, the content of polymerizable monomer (A-2) is preferably 20 to 55 mass%, more preferably 25 to 50 mass%, even more preferably 30 to 45 mass% in a total amount of the dental curable composition.

The content of polymerizable monomer (A-2) is preferably 20 to 80 mass%, more preferably 25 to 75 mass%, even more preferably 30 to 70 mass% in total 100 parts by mass of polymerizable compound (A).

In view of the curability and operability of the composition, and the hardness and toughness of the cured product, the mass ratio (A-1):(A-2) of the content of urethane (meth)acrylate oligomer (A-1) and the content of polymerizable monomer (A-2) is preferably 100:5 to 100:500, more preferably 100:10 to 100:300, even more preferably 100:20 to 100:200.

A certain preferred embodiment is, for example, a dental curable composition in which the polymerizable compound (A) comprises a polymerizable monomer (A-2), and the polymerizable monomer (A-2) comprises an adhesive monomer (a-4) having at least one acidic group per molecule (hereinafter, also referred to as "adhesive monomer (a-4) having an acidic group"). With an adhesive monomer (a-4) having an acidic group, a stronger bond can be formed at the interface between the base material and the cured product of the composition forming a resin layer on the base material.

A dental adhesive composition (G) comprising an adhesive monomer (a-4) having an acidic group (hereinafter, referred to as "adhesive composition (G)"), different from a dental curable composition of the present invention, may be used with a dental curable composition of the present invention. A certain preferred embodiment is, for example, a dental kit that comprises a dental curable composition of the present invention, and a dental adhesive composition (G) comprising an adhesive monomer (a-4) having an acidic group. The dental curable composition of the present invention used for the dental kit may or may not comprise an adhesive monomer (a-4) having an acidic group. In the following, a dental

adhesive composition (G) will be called a "primer composition (G)" when it contains a volatile organic solvent (H) in addition to the adhesive monomer (a-4) having an acidic group. The dental adhesive composition (G) used for the dental kit may be a commercially available product (for example, such as Clearfil® Ceramic Primer Plus manufactured by Kuraray Noritake Dental Inc. under this trade name). The adhesive composition (G) comprising an adhesive monomer (a-4) having an acidic group enables formation of a stronger bond at the interface between the base material and the cured product of the composition forming a resin layer on the base material.

The adhesive monomer (a-4) having an acidic group is a polymerizable monomer having an acidic group such as a phosphoric acid group, a phosphonic acid group, a pyrophosphoric acid group, a thiophosphoric acid group, a carboxylic acid group, or a sulfonic acid group. Specific examples are as follows.

Examples of the phosphoric acid group-containing polymerizable monomer include 2-(meth)acryloyloxyethyl dihydrogen phosphate, 10-(meth)acryloyloxydecyl dihydrogen phosphate, 20-(meth)acryloyloxyeicosyl dihydrogen phosphate, 1,3-di(meth)acryloyloxypropyl-2-dihydrogen phosphate, 2-(meth)acryloyloxyethylphenyl phosphoric acid, 2-(meth)acryloyloxyethyl-2'-bromoethyl phosphoric acid, (meth)acryloyloxyethylphenyl phosphonate, and acid chlorides of these.

Examples of the phosphonic acid group-containing polymerizable monomer include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, 10-(meth)acryloyloxydecylphosphonoacetate, and acid chlorides of these.

Examples of the pyrophosphoric acid group-containing polymerizable monomer include di[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides of these.

Examples of the thiophosphoric acid group-containing polymerizable monomer include 2-(meth)acryloyloxyethyl dihydrogen dithiophosphate, 10-(meth)acryloyloxydecyl dihydrogen thiophosphate, and acid chlorides of these.

Examples of the carboxylic acid group-containing polymerizable monomer include (meth)acrylic acid, mono(2-(meth)acryloyloxyethyl)succinate, mono(2-(meth)acryloyloxyethyl)isophthalate, N-(meth)acryloyl-5-aminosalicylic acid, 4-vinyl benzoic acid, 4-(meth)acryloyloxyethoxycarbonyl phthalic acid, 4-(meth)acryloyloxyethoxycarbonyl phthalic acid anhydride, 5-(meth)acryloylaminopentyl carboxylic acid, 11-(meth)acryloyloxyundecane-1,1-dicarboxylic acid, and acid chlorides of these. Examples of the sulfonic acid group-containing polymerizable monomer include p-styrenesulfonic acid.

In view of excellence of adhesive properties at the interface, preferred among these compounds are phosphoric acid group-containing polymerizable monomers or phosphonic acid group-containing polymerizable monomers, particularly 10-(meth)acryloyloxydecyl dihydrogen phosphate, 2-(meth)acryloyloxyethylphenyl phosphoric acid, and 2-(meth)acryloyloxyethylphenyl phosphonate. These adhesive monomers (a-4) having an acidic group may be used alone, or two or more thereof may be used in combination.

The content of the adhesive monomer (a-4) having an acidic group in the adhesive composition (G) is not particularly limited, and is preferably 0.1 to 10 mass%, more preferably 1 to 5 mass%, even more preferably 1 to 3 mass% in a total amount of the adhesive composition (G).

When the dental curable composition comprises an adhesive monomer (a-4) having an acidic group, the content of the adhesive monomer (a-4) having an acidic group is preferably 1 to 35 mass%, more preferably 2 to 30 mass%, even more preferably 5 to 25 mass% in total 100 mass% of polymerizable compound (A).

<Polymerization Accelerator (B)>

In a dental curable composition of the present invention, the polymerization accelerator (B) comprises a polyfunctional thiol (B-1) having two or more mercapto groups per molecule.

A certain embodiment is, for example, a dental curable composition in which a polyfunctional thiol (B-1) having two or more mercapto groups per molecule is the sole mercapto group-containing compound in the polymerization accelerator (B). In such an embodiment, the polymerization accelerator (B) may comprise a polymerization accelerator (B-2) other than the polyfunctional thiol (B-1).

Another certain embodiment is, for example, a dental curable composition in which the polymerization accelerator (B) is a polyfunctional thiol (B-1) having two or more mercapto groups per molecule.

Yet another certain embodiment is, for example, a dental curable composition that does not comprise a monofunctional thiol compound.

· Polyfunctional Thiol (B-1)

The following describes the polyfunctional thiol (B-1) having two or more mercapto groups per molecule (hereinafter, also referred to as "polyfunctional thiol (B-1)") included in the present invention.

The polyfunctional thiol (B-1) used in the present invention is a compound having two or more mercapto groups per molecule. When combined with the polymerization initiator (D) (described later) in the presence of the polymerizable compound (A), the polyfunctional thiol (B-1) can improve the crosslink density by accelerating curing of the polymerizable compound (A), producing an even stronger cured product with higher impact resistance.

Enhanced curability is probably a result of the accelerated photocrosslinking reaction due to the use of the polyfunctional thiol (B-1), rather than a monofunctional thiol. This provides the desired pencil hardness to the cured product.

The cured product of the dental curable composition also exhibits superior abrasion resistance due to its high impact resistance. This can be a contributing factor of enhanced gloss retention and fluorescence durability. Additionally, because of accelerated curing, the polymerization initiator (D) can be used in reduced amounts, making it possible to reduce shrinkage during cure.

Moreover, the multiple mercapto groups of the polyfunctional thiol (B-1) form hydrogen bonds with the OH groups on the ceramic surface representing the base material, making it possible to enhance the adhesion between the resin layer and the base material, and increase the refractive index by the presence of sulfur atoms in the molecule. This allows the polyfunctional thiol (B-1), when used with the fluorescent agent (C), to enable an even greater reduction of shade changes when the base material is coated with the dental curable composition, while imparting fluorescence to the base material.

This outstanding effect to reduce shade changes in the base material while imparting fluorescence to the base material stems from the multiple mercapto groups of the polyfunctional thiol (B-1), and cannot be achieved with monofunctional thiol compounds.

While the specific reason remains unclear, the multiple mercapto groups on the polyfunctional thiol (B-1) appear to extendedly mitigate the decline in fluorescence resulting from the contact between fluorescent agent (C) and filler (E), probably by forming hydrogen bonds with the OH groups present on the surface of the filler (E) described below. By incorporating the polyfunctional thiol (B-1), a dental curable composition of the present invention can impart fluorescence to the base material lacking a fluorescent agent when built up on the base material in coating applications involving dental prostheses such as zirconia.

The polyfunctional thiol (B-1) is not particularly limited, as long as it is a compound having two or more mercapto groups per molecule. In the context of polyfunctional thiol (B-1), "polyfunctional" means having two or more mercapto groups per molecule. Specific examples include compounds having two mercapto groups per molecule, compounds having three mercapto groups per molecule, compounds having four mercapto groups per molecule, and compounds having six mercapto groups per molecule. In view of excellence of curability improving effect and shorter curing time, preferred are polyfunctional thiols having 2 to 4 mercapto groups per molecule, more preferably polyfunctional thiols having three mercapto groups per molecule, and polyfunctional thiols having four mercapto groups per molecule.

Examples of compounds having two mercapto groups per molecule include:

compounds having two primary mercapto groups per molecule, such as butanediol bis(2-mercaptoacetate), hexanediol bis(2-mercaptoacetate), ethanediol bis(2-mercaptoacetate), butanediol bis(3-mercaptopropionate), 2,2'-(ethylenedithio)diethanethiol, ethylene glycol bis(3-mercapto-2-methylpropionate), ethylene glycol bis(3-mercaptopropionate), propylene glycol bis(3-mercaptopropionate), diethylene glycol bis(3-mercaptopropionate), octanediol bis(3-mercaptopropionate), propylene glycol bis(3-mercapto-2-methylpropionate), diethylene glycol bis(3-mercapto-2-methylpropionate), butanediol bis(3-mercapto-2-methylpropionate), octanediol bis(3-mercapto-2-methylpropionate), and bis(3-mercapto-2-methylpropyl)phthalate:

compounds having two secondary mercapto groups per molecule, such as 1,4-bis(3-mercaptobutyryloxy)butane, bis(1-mercaptoethyl)phthalate, bis(2-mercaptopropyl)phthalate, bis(3-mercaptobutyl)phthalate, ethylene glycol bis(3-mercaptobutyrate), propylene glycol bis(3-mercaptobutyrate), diethylene glycol bis(3-mercaptobutyrate), butanediol bis(3-mercaptobutyrate), octanediol bis(3-mercaptobutyrate), ethylene glycol bis(2-mercaptopropionate), propylene glycol bis(2-mercaptopropionate), diethylene glycol bis(2-mercaptopropionate), butanediol bis(2-mercaptopropionate), octanediol bis(2-mercaptopropionate), ethylene glycol bis(4-mercaptovalerate), propylene glycol bis(4-mercaptoisovalerate), diethylene glycol bis(4-mercaptovalerate), butanediol bis(4-mercaptovalerate), octanediol bis(4-mercaptovalerate), ethylene glycol bis(3-mercaptovalerate), propylene glycol bis(3-mercaptovalerate), diethylene glycol bis(3-mercaptovalerate), butanediol bis(3-mercaptovalerate), and octanediol bis(3-mercaptovalerate); and

compounds having two tertiary mercapto groups per molecule, such as ethylene glycol bis(2-mercaptoisobutyrate), propylene glycol bis(2-mercaptoisobutyrate), diethylene glycol bis(2-mercaptoisobutyrate), butanediol bis(2-mercaptoisobutyrate), and octanediol bis(2-mercaptoisobutyrate).

In this specification, "primary mercapto group" means a mercapto group attached to a primary carbon atom, "secondary mercapto group" means a mercapto group attached to a secondary carbon atom, and "tertiary mercapto

group" means a mercapto group attached to a tertiary carbon atom. A mercapto group being attached to a secondary carbon atom or tertiary carbon atom means that the polyfunctional thiol (B-1) has secondary mercapto groups or tertiary mercapto groups.

Examples of compounds having three mercapto groups per molecule include:

compounds having three primary mercapto groups per molecule, such as trimethylolpropane tris(2-mercaptoacetate), trimethylolpropane tris(3-mercaptopropionate), trimethylolpropane tris(3-mercapto-2-methylpropionate), and 3-(3-mercapto-propoxy)-2,2-bis-(3-mercapto-propoxymethyl)-propan-1-ol;
compounds having three secondary mercapto groups per molecule, such as trimethylolpropane tris(3-mercaptobutyrate), trimethylolpropane tris(2-mercaptopropionate), trimethylolpropane tris(4-mercaptovalerate), trimethylolpropane tris(3-mercaptovalerate), and 1,3,5-tris(3-mercaptobutyloxyethyl)-1,3,5-triazine-2,4,6(1H,3H,5H)-trione; and
compounds having three tertiary mercapto groups per molecule, such as trimethylolpropane tris(2-mercaptoisobutyrate).

Examples of compounds having four mercapto groups per molecule include:

compounds having four primary mercapto groups per molecule, such as pentaerythritol tetrakis(2-mercaptoacetate), and pentaerythritol tetrakis(3-mercaptopropionate);
compounds having four secondary mercapto groups per molecule, such as dipentaerythritol hexakis(3-mercaptobutyrate), pentaerythritol tetrakis(2-mercaptopropionate), pentaerythritol tetrakis(3-mercaptobutyrate), pentaerythritol tetrakis(3-mercapto-2-propionate), pentaerythritol tetrakis(4-mercaptovalerate), and pentaerythritol tetrakis(3-mercaptovalerate); and
compounds having four tertiary mercapto groups per molecule, such as pentaerythritol tetrakis(2-mercaptoisobutyrate).

Examples of compounds having six mercapto groups per molecule include:

compounds having six primary mercapto groups per molecule, such as dipentaerythritol hexakis(3-mercapto-2-methylpropionate);
compounds having six secondary mercapto groups per molecule, such as dipentaerythritol hexakis(3-mercaptobutyrate), dipentaerythritol hexakis(2-mercaptopropionate), dipentaerythritol hexakis(4-mercaptovalerate), and dipentaerythritol hexakis(3-mercaptovalerate); and
compounds having six tertiary mercapto groups per molecule, such as dipentaerythritol hexakis(2-mercaptoisobutyrate).

Preferred among these polyfunctional thiols (B-1) are compounds having secondary or tertiary mercapto groups. In view of suppressing the unpleasant odor of the curable composition, and providing improved storage stability, more preferred are compounds having no primary mercapto group, and in which the number of secondary mercapto groups and the number of tertiary mercapto groups are two or more in total.

For example, preferred as polyfunctional thiols (B-1) are compounds having two secondary mercapto groups per molecule, compounds having two tertiary mercapto groups per molecule, compounds having three secondary mercapto groups per molecule, compounds having three tertiary mercapto groups per molecule, compounds having four secondary mercapto groups per molecule, compounds having four tertiary mercapto groups per molecule, compounds having six secondary mercapto groups per molecule, and compounds having six tertiary mercapto groups per molecule, more preferably compounds having two secondary mercapto groups per molecule, compounds having two tertiary mercapto groups per molecule, compounds having three secondary mercapto groups per molecule, compounds having three tertiary mercapto groups per molecule, compounds having four secondary mercapto groups per molecule, and compounds having four tertiary mercapto groups per molecule, even more preferably compounds having three secondary mercapto groups per molecule, compounds having three tertiary mercapto groups per molecule, compounds having four secondary mercapto groups per molecule, and compounds having four tertiary mercapto groups per molecule.

The polyfunctional thiol (B-1) is preferably 3-(3-mercapto-propoxy)-2,2-bis-(3-mercapto-propoxymethyl)-propan-1-ol, pentaerythritol tetrakis(3-mercaptobutyrate), or 1,3,5-tris(3-mercaptobutyloxyethyl)-1,3,5-triazine-2,4,6(1H,3H,5H)-trione, particularly preferably pentaerythritol tetrakis(3-mercaptobutyrate), or 1,3,5-tris(3-mercaptobutyloxyethyl)-1,3,5-triazine-2,4,6(1H,3H,5H)-trione.

The polyfunctional thiol (B-1) may be used alone, or two or more thereof may be used in combination. The molecular weight of polyfunctional thiol (B-1) is not particularly limited. However, in view of improving the curability of the curable composition, the polyfunctional thiol (B-1) has a molecular weight of preferably 200 to 1,000.

The polyfunctional thiol (B-1) is also readily available as a commercially available product. Examples of such

commercially available products of polyfunctional thiols having two or more mercapto groups per molecule include 1,4-bis(3-mercaptobutyryloxy)butane (KarenzMT® BD1 manufactured by Showa Denko K.K. under this trade name), pentaerythritol tetrakis(3-mercaptobutyrate) (KarenzMT® PE1 manufactured by Showa Denko K.K. under this trade name), 1,3,5-tris(3-mercaptobutyryloxyethyl)-1,3,5-triazine-2,4,6(1H,3H,5H)-trione (KarenzMT® NR1 manufactured by Showa Denko K.K. under this trade name), and trimethylolpropane tris(3-mercaptobutyrate) (TPMB manufactured by Showa Denko K.K. under this trade name).

In view of the gloss of the cured product, the polyfunctional thiol (B-1) has a refractive index of preferably 1.40 or greater, more preferably 1.45 or greater, even more preferably 1.50 or greater.

The refractive index of polyfunctional thiol (B-1) can be measured with an Abbe refractometer. The refractive index can be measured according to JIS K 0062:1992 with some modification, specifically, by a liquid immersion method at 23°C using an Abbe refractometer, with the sodium D-line serving as the light source. As for the liquid, different liquids with varying refractive indices are prepared by combining two or more liquids whose refractive indices are similar to the presumed refractive index of the sample filler (polyfunctional thiol (B-1)). The sample is suspended in each liquid in a 23°C atmosphere, and the liquid appearing most transparent as observed by the naked eye is selected. By regarding the refractive index of this liquid as the refractive index of the sample, the liquid's refractive index is measured with an Abbe refractometer. Examples of usable liquids include diiodomethane with sulfur dissolved in it, 1-bromonaphthalene, methyl salicylate, dimethylformamide, and 1-pentanol.

In view of enhancing curability, it is preferable to determine the content of polymerizable compound (A) and polyfunctional thiol (B-1) based on the number of unsaturated groups in the polymerizable compound (A) and the number of mercapto groups in the polyfunctional thiol (B-1).

In certain preferred embodiments, the ratio of the number of unsaturated groups in the polymerizable compound (A) with respect to the number of mercapto groups in the polyfunctional thiol (B-1) ([number of unsaturated groups] / [number of mercapto groups]) ranges preferably from 0.25 to 4.

Because the reaction between unsaturated groups and mercapto groups is equivalent, an excess of unsaturated groups compared to mercapto groups may easily lead to the generation of radicals through heat, resulting in degradation of the cured product.

Conversely, an excess of mercapto groups compared to unsaturated groups may cause the release of polyfunctional thiol (B-1) due to the decomposition of the cured product from heat or humid heat, resulting in an unpleasant odor.

From the same perspective mentioned above, as well as the improvement of adhesive properties to the base material, the content of polyfunctional thiol (B-1) is preferably 5 to 40 mass%, more preferably 10 to 35 mass%, even more preferably 15 to 30 mass% in a total amount of the dental curable composition. With the content of the polyfunctional thiol (B-1) in the curable composition falling in these ranges, it is possible to reduce odor before and after the curing of the curable composition, and provide a cured product having enhanced curability and improved adhesive properties to the base material.

In this specification, the number of unsaturated groups in polymerizable compound (A) means the total number (in moles) of the unsaturated groups in all compounds belonging to polymerizable compound (A), and the number of mercapto groups in polyfunctional thiol (B-1) means the total number (in moles) of the mercapto groups in all compounds belonging to polyfunctional thiol (B-1).

<Fluorescent Agent (C)>

The fluorescent agent (C) included in the present invention can be selected from fluorescent agents that are commonly available, and may be one or multiple fluorescent agents capable of emitting fluorescence at a given wavelength of light. Particularly preferred for use are fluorescent agents used in dentistry.

The fluorescent agent (C) may be, for example, an inorganic fluorescent agent (C-1) or an organic fluorescent agent (C-2). The fluorescent agent (C) may be used alone, or two or more thereof may be used in appropriate combinations. In view of more evenly imparting fluorescence, the fluorescent agent (C) preferably comprises an organic fluorescent agent (C-2).

The inorganic fluorescent agent (C-1) may be those containing metallic elements, for example. Examples of the metallic elements include Ga, Bi, Ce, Nd, Sm, Eu, Gd, Tb, Dy, and Tm. The fluorescent agent may comprise one such metallic element alone, or may comprise two or more metallic elements. Preferred among these metallic elements are Ga, Bi, Eu, Gd, and Tm, more preferably Bi and Eu.

Examples of the fluorescent agent include oxides, hydroxides, acetates, and nitrates of the foregoing metallic elements. The fluorescent agent may be, for example, $Y_2SiO_5$:Ce, $Y_2SiO_5$:Tb, (Y,Gd,Eu)$BO_3$, $Y_2O_3$:Eu, YAG:Ce, $ZnGa_2O_4$:Zn, or $BaMgAl_{10}O_{17}$:Eu.

In view of exhibiting fluorescence comparable to that of human natural teeth, the content of inorganic fluorescent agent (C-1) is preferably 0.01 to 30 mass%, more preferably 0.1 to 20 mass%, even more preferably 1 to 10 mass% in

a total amount of the dental curable composition.

When the content of inorganic fluorescent agent (C-1) is less than 0.01 mass%, inadequate fluorescence may result compared to the fluorescence of human natural teeth, leading to poor aesthetics. When the content of inorganic fluorescent agent (C-1) is higher than 30 mass%, it may result in poor dispersibility, leading to uneven fluorescence and an unnatural appearance.

The mass ratio (B-1):(C-1) of the content of polyfunctional thiol (B-1) and the content of inorganic fluorescent agent (C-1) is preferably 100:0.001 to 100:300. In view of the capability to reduce shade changes in the base material while imparting fluorescence to the base material, as well as providing excellent abrasion resistance, gloss retention, and fluorescence durability in the cured product, the mass ratio is more preferably 100:0.01 to 100:200. In view of enhancing the effect to reduce shade changes in the base material, and providing even superior gloss retention and fluorescence durability, the mass ratio is even more preferably 100:0.1 to 100:100.

The mass ratio (B-1):(C) of the content of polyfunctional thiol (B-1) and the content of fluorescent agent (C) (a total content of inorganic fluorescent agent (C-1) and organic fluorescent agent (C-2)) is preferably 100:0.001 to 100:600. In view of the capability to reduce shade changes in the base material while imparting fluorescence to the base material, as well as providing excellent abrasion resistance, gloss retention, and fluorescence durability in the cured product, the mass ratio is more preferably 100:0.01 to 100:300. In view of enhancing the effect to reduce shade changes in the base material, and providing even superior gloss retention and fluorescence durability, the mass ratio is even more preferably 100:0.1 to 100:200.

Examples of the organic fluorescent agent (C-2) include phthalic acid derivatives (e.g., diethyl 2,5-dihydroxyterephthalate, and o-phthalaldehyde), thiophene derivatives (2,5-bis(5'-t-butylbenzooxazolyl-2')thiophene, 2,5-bis(6,6'-bis(tert-butyl)-benzoxazol-2-yl)thiophene), naphthalene derivatives (1,4-bis(2-benzooxazolyl)naphthalene), coumarin derivatives (3-phenyl-7-(4-methyl-5-phenyl-1,2,3-triazol-2-yl)coumarin, 3-phenyl-7-(2H-naphtho[1,2-d]-triazol-2-yl)coumarin), naphthalimide derivatives (N-methyl-5-methoxynaphthalimide), stilbene derivatives (4,4'-bis(diphenyltriazinyl)stilbene, 4,4'-bis(benzooxazolyl-2-yl)stilbene), benzothiazole derivatives, and quinazolinone derivatives. Preferred among these are phthalic acid derivatives, and thiophene derivatives, more preferably phthalic acid derivatives.

Examples of the phthalic acid derivatives include compounds represented by the following formula (1),

[Chem. 1]

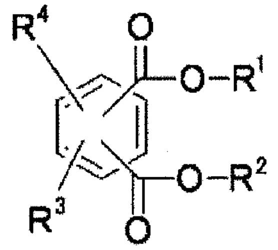

(1)

wherein $R^1$ and $R^2$ are each independently an alkyl group, $R^3$ is a hydrogen atom, an amino group, or a hydroxyl group, and $R^4$ is an amino group or a hydroxyl group.

The alkyl groups represented by $R^1$ and $R^2$ are preferably alkyl groups having 1 to 3 carbon atoms, such as methyl groups, ethyl groups, n-propyl groups, and isopropyl groups, more preferably methyl groups and ethyl groups.

Examples of compounds represented by formula (1) include dimethyl 2,5-dihydroxyterephthalate, diethyl 2,5-dihydroxyterephthalate, dimethyl 2-aminoterephthalate, and diethyl 2-aminoterephthalate. Particularly preferred is diethyl 2,5-dihydroxyterephthalate.

In view of exhibiting fluorescence comparable to that of human natural teeth, the content of organic fluorescent agent (C-2) is preferably 0.001 to 10 mass%, more preferably 0.01 to 8 mass%, even more preferably 0.05 to 5 mass%. When the content of organic fluorescent agent (C-2) is less than 0.001 mass%, inadequate fluorescence may result compared to the fluorescence of human natural teeth, leading to poor aesthetics. When the content of organic fluorescent agent (C-2) is higher than 10 mass%, it may result in excessive fluorescence compared to the fluorescence of human natural teeth, leading to an unnatural appearance.

The mass ratio (B-1):(C-2) of the content of polyfunctional thiol (B-1) and the content of organic fluorescent agent (C-2) is preferably 100:0.001 to 100:600. In view of the capability to reduce shade changes in the base material while imparting fluorescence to the base material, as well as providing excellent abrasion resistance, gloss retention, and fluorescence durability in the cured product, the mass ratio is more preferably 100:0.01 to 100:300. In view of enhancing the effect to reduce shade changes in the base material, and providing even superior gloss retention and fluorescence durability, the mass ratio is even more preferably 100:0.1 to 100:200.

<Polymerization Initiator (D)>

The following describes the polymerization initiator (D) included in the present invention.

The polymerization initiator (D) can be selected from polymerization initiators that are commonly available. Particularly preferred for use are polymerization initiators used in dentistry.

Examples of the polymerization initiator (D) include photopolymerization initiators (D-1) and chemical polymerization initiators (D-2).

The polymerization initiator (D) may be used alone, or two or more thereof may be used in appropriate combinations.

In view of surface curability, the polymerization initiator (D) preferably comprises a photopolymerization initiator (D-1).

Examples of the photopolymerization initiator (D-1) include α-diketones, ketals, thioxanthones, acylphosphine oxides, and α-aminoacetophenones.

Examples of the α-diketones include dl-camphorquinone, benzyl, and 2,3-pentanedione.

Examples of the ketals include benzyl dimethyl ketal, and benzyl diethyl ketal.

Examples of the thioxanthones include 2-chlorothioxanthone, and 2,4-diethylthioxanthone.

Examples of the acylphosphine oxides include

2,4,6-trimethylbenzoyldiphenylphosphine oxide, bis(2,4,6-trimethylbenzoyl)phenylphosphine oxide, bis(benzoyl)phenylphosphine oxide, bis(2,6-dimethoxybenzoyl)phenylphosphine oxide, tris(2,4-dimethylbenzoyl)phosphine oxide, tris(2-methoxybenzoyl)phosphine oxide, 2,6-dimethoxybenzoyldiphenylphosphine oxide, 2,6-dichlorobenzoyldiphenylphosphine oxide,

2,3,5,6-tetramethylbenzoyldiphenylphosphine oxide, benzoyl di(2,6-dimethylphenyl)phosphonate, 2,4,6-trimethylbenzoylethoxyphenylphosphine oxide, and the water-soluble acylphosphine oxide compounds disclosed in JP H03-57916 B.

Examples of the α-aminoacetophenones include 2-benzyl-dimethylamino-1-(4-morpholinophenyl)-1-butanone, 2-benzyl-diethylamino-1-(4-morpholinophenyl)-1-butanone, 2-benzyl-dimethylamino-1-(4-morpholinophenyl)-1-propanone, 2-benzyl-diethylamino-1-(4-morpholinophenyl)-1-propanone, 2-benzyl-dimethylamino-1-(4-morpholinophenyl)-1-pentanone, and 2-benzyl-diethylamino-1-(4-morpholinophenyl)-1-pentanone.

The photopolymerization initiator (D-1) may be used alone, or two or more thereof may be used in combination. The content of photopolymerization initiator (D-1) is preferably 0.01 to 15 parts by mass, more preferably 0.05 to 12 parts by mass, even more preferably 0.1 to 10 parts by mass with respect to total 100 parts by mass of polymerizable compound (A).

The photopolymerization initiator (D-1) may be used alone. Alternatively, in order to accelerate curing under light, the photopolymerization initiator (D-1) may be used with polymerization accelerators (B-2) (hereinafter, also referred to as "additional polymerization accelerators (B-2)"), such as tertiary amines, aldehydes, and monofunctional thiols, other than polyfunctional thiol (B-1).

Examples of the tertiary amines include aliphatic tertiary amines such as 2-(dimethylamino)ethyl (meth)acrylate, N,N-bis[(meth)acryloyloxyethyl]-N-methylamine, N-methyldiethanolamine di(meth)acrylate, N-ethyldiethanolamine di(meth)acrylate, triethanolamine mono(meth)acrylate, triethanolamine di(meth)acrylate, triethanolamine tri(meth)acrylate, triethanolamine, trimethylamine, triethylamine, tributylamine, N-methyldiethanolamine, N-ethyldiethanolamine, and N-n-butyldiethanolamine; and aromatic tertiary amines such as ethyl 4-(N,N-dimethylamino)benzoate, butyl 4-(N,N-dimethylamino)benzoate, butoxyethyl 4-(N,N-dimethylamino)benzoate, 4-(N,N-dimethylamino)benzophenone, and N,N-di(2-hydroxyethyl)-p-toluidine.

Examples of the aldehydes include dimethylaminobenzaldehyde, and terephthalaldehyde.

Examples of the monofunctional thiols include compounds having one mercapto group per molecule, such as 2-mercaptobenzoxazole, decanethiol, 3-mercaptopropyltrimethoxysilane, and thiobenzoic acid.

The additional polymerization accelerator (B-2) may be used alone, or two or more thereof may optionally be used in combination. The content of additional polymerization accelerator (B-2) is preferably 0.01 to 15 parts by mass, more preferably 0.05 to 12 parts by mass, even more preferably 0.1 to 10 parts by mass with respect to total 100 parts by mass of polymerizable compound (A).

The chemical polymerization initiator (D-2) is preferably a redox polymerization initiator composed of an oxidizing agent and a reducing agent. When using a redox polymerization initiator, the polymerizable compound (A) must be packed in at least two to separate the oxidizing agent and the reducing agent.

Examples of the oxidizing agent of the redox polymerization initiator include organic peroxides such as diacyl peroxides, peroxyesters, dialkyl peroxides, peroxyketals, ketone peroxides, and hydroperoxides.

Specific examples of the diacyl peroxides include benzoyl peroxide, 2,4-dichlorobenzoyl peroxide, and m-toluoyl

peroxide.

Specific examples of the peroxyesters include t-butyl peroxybenzoate, bis-t-butyl peroxyisophthalate, 2,5-dimethyl-2,5-bis(benzoylperoxy)hexane, t-butyl peroxy-2-ethylhexanoate, and t-butyl peroxyisopropyl carbonate.

Specific examples of the dialkyl peroxides include dicumyl peroxide, di-t-butyl peroxide, and lauroyl peroxide.

Specific examples of the peroxyketals include 1,1-bis(t-butylperoxy)-3,3,5-trimethylcyclohexane.

Specific examples of the ketone peroxides include methyl ethyl ketone peroxide, cyclohexanone peroxide, and methyl acetoacetate peroxide.

Specific examples of the hydroperoxides include t-butyl hydroperoxide, cumene hydroperoxide, and p-diisopropyl benzene peroxide.

Examples of the reducing agent of the redox polymerization initiator include aromatic tertiary amines, aliphatic tertiary amines, and sulfinic acid and its salts.

Examples of the aromatic tertiary amines include N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-di(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-dibutylaniline, ethyl 4-(N,N-dimethylamino)benzoate, n-butoxyethyl 4-(N,N-dimethylamino)benzoate, (2-methacryloyloxy)ethyl 4-(N,N-dimethylamino)benzoate, and 4-(N,N-dimethylamino)benzophenone.

Examples of the aliphatic tertiary amines include trimethylamine, triethylamine, N-methyldiethanolamine, N-ethyldiethanolamine, N-n-butyldiethanolamine, N-lauryldiethanolamine, triethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, and triethanolamine trimethacrylate.

Examples of sulfinic acid and its salts include benzenesulfinic acid, sodium benzenesulfinate, potassium benzenesulfinate, calcium benzenesulfinate, lithium benzenesulfinate, p-toluenesulfinic acid, sodium p-toluenesulfinate, potassium p-toluenesulfinate, calcium p-toluenesulfinate, lithium p-toluenesulfinate, 2,4,6-trimethylbenzenesulfinic acid, sodium 2,4,6-trimethylbenzenesulfinate, potassium 2,4,6-trimethylbenzenesulfinate, calcium 2,4,6-trimethylbenzenesulfinate, lithium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, calcium 2,4,6-triethylbenzenesulfinate, lithium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, calcium 2,4,6-triisopropylbenzenesulfinate, and lithium 2,4,6-triisopropylbenzenesulfinate.

The oxidizing agent and reducing agent each may be used alone, or two or more thereof may optionally be used in combination. The content of the oxidizing agent and the content of the reducing agent are both preferably 0.01 to 15 parts by mass, more preferably 0.05 to 12 parts by mass, even more preferably 0.1 to 10 parts by mass relative to total 100 parts by mass of polymerizable compound (A).

<Filler (E)>

A dental curable composition of the present invention may comprise a filler, in order to enhance mechanical strength and abrasion resistance, and to adjust properties such as coatability and flowability during application. The filler (E) may be an inorganic filler, an organic filler, or an inorganic/organic composite filler.

Examples of the inorganic filler include minerals containing silica as the base material, such as silica, kaolin, clay, *ummo,* and mica; and ceramics or glass containing compounds such as $Al_2O_3$, $B_2O_3$, $TiO_2$, $ZrO_2$, BaO, $La_2O_3$, $SrO_2$, CaO, or $P_2O_5$, in addition to silica serving as the base material.

Specific examples of such glass include lanthanum glass, barium glass, strontium glass, soda glass, lithium borosilicate glass, aluminosilicate glass, barium boroaluminosilicate glass, strontium boroaluminosilicate glass, zinc glass, fluoroaluminosilicate glass, borosilicate glass, calcium fluoroaluminosilicate glass, strontium fluoroaluminosilicate glass, barium fluoroaluminosilicate glass, strontium calcium fluoroaluminosilicate glass, and bioglass. Other examples of the inorganic filler include crystal quartz, hydroxyapatite, alumina, titania, yttrium oxide, zirconia, calcium phosphate, barium sulfate, aluminum hydroxide, sodium fluoride, potassium fluoride, sodium monofluorophosphate, lithium fluoride, calcium fluoride, ytterbium fluoride, and yttrium fluoride. Preferred among these are silica, alumina, and titania, more preferably silica.

In view of enhancing the strength of the cured product, the inorganic filler has a particle diameter of preferably 100 nm or less, more preferably 90 nm or less, even more preferably 80 nm or less in terms of an average primary particle diameter. In view of the viscosity of the composition, the average primary particle diameter is preferably 15 nm or more, more preferably 20 nm or more, even more preferably 25 nm or more. The average primary particle diameter may fall in any combinations of these ranges. For example, the average primary particle diameter is preferably 15 to 100 nm, more preferably 20 to 90 nm, even more preferably 25 to 80 nm.

The average particle diameter of inorganic filler means the average particle diameter before surface treatment when the inorganic filler is surface-treated as described below.

In view of enhancing the affinity for the polymerizable compound (A) and/or polyfunctional thiol (B-1), and improving the antifouling properties, it is preferable that the inorganic filler be hydrophobic. The inorganic filler has a methanol hydrophobicity of preferably 15% or more, more preferably 20% or more, even more preferably 25% or more.

A certain embodiment is, for example, a dental curable composition in which the filler (E) comprises a hydrophobic silica (E-1) as an inorganic filler.

The methanol hydrophobicity is measured as follows. An amount of 0.1 g of a sample is weighed and placed in a 200 mL beaker, followed by addition of 50 mL of ion-exchange water, and stirring with a magnetic stirrer. Subsequently, methanol is added dropwise using a burette, at a rate of about 2 mL every 10 seconds, until the sample floating on the liquid surface completely disappears, indicating the end point. The methanol hydrophobicity is then calculated using the following formula.

$$\text{Methanol hydrophobicity (\%)} = [\text{volume of titrant} / (\text{volume of titrant} + 50)] \times 100$$

In view of improving affinity by enhancing its interaction with polysiloxane (F), the inorganic filler is preferably one that has been surface-treated with a surface treatment agent. This not only helps prevent aggregation of inorganic fillers, leading to a reduction in the viscosity of the composition, but also improves the strength and abrasion resistance of the cured product.

The surface treatment agent may be, for example, a silane coupling agent.

Examples of the silane coupling agent include, but are not particularly limited to, methyltrimethoxysilane, dimethyldimethoxysilane, phenyltrimethoxysilane, diphenyldimethoxysilane, methyltriethoxysilane, dimethyldiethoxysilane, phenyltriethoxysilane, diphenyldiethoxysilane, isobutyltrimethoxysilane, vinyltrimethoxysilane, vinyltriethoxysilane, vinyltris(2-methoxyethoxy)silane, 3,3,3-trifluoropropyltrimethoxysilane, methyl-3,3,3-trifluoropropyldimethoxysilane, 2-(3,4-epoxycyclohexyl)ethyltrimethoxysilane, 3-glycidoxypropyltrimethoxysilane, 3-glycidoxypropylmethyldiethoxysilane, 3-glycidoxypropyltriethoxysilane, 3-methacryloyloxypropylmethyldimethoxysilane, γ-methacηloyloxypropylmethyldiethoxysilane, N-β(aminoethyl)γ-aminopropylmethyldimethoxysilane, N-β(aminoethyl)γ-aminopropyltrimethoxysilane, N-β(aminoethyl)γ-aminopropyltriethoxysilane, 3-aminopropyltrimethoxysilane, 3-aminopropyltriethoxysilane, N-phenyl-γ-aminopropyltrimethoxysilane, 3-mercaptopropyltrimethoxysilane, trimethylsilanol, methyltrichlorosilane, methyldichlorosilane, dimethyldichlorosilane, trimethylchlorosilane, phenyltrichlorosilane, diphenyldichlorosilane, vinyltrichlorosilane, trimethylbromosilane, diethylsilane, vinyltriacetoxysilane, ω-(meth)acryloyloxyalkyltrimethoxysilane (3 to 12 carbon atoms between the (meth)acryloyloxy group and the silicon atom; e.g., 3-methacryloyloxypropyltrimethoxysilane), ω-(meth)acryloyloxyalkyltriethoxysilane (3 to 12 carbon atoms between the (meth)acryloyloxy group and the silicon atom; e.g., 3-methacryloyloxypropyltriethoxysilane).

The particle shape is not particularly limited, and the surface treatment agent may be used as a powder of irregularly shaped particles or spherical particles.

The inorganic particles may be commercially available products. Examples include:

Aerosil® OX50, Aerosil® 50, Aerosil® 130, Aerosil® 200, Aerosil® 380, Aerosil® MOX80, Aerosil® R972, Aerosil® RY50, Aerosil® NAX50, Aerosil® NX90G, Aeroxide® AluC, Aeroxide® $TiO_2$ P25, VP Zirconium Oxide 3-YSZ, and VP Zirconium Oxide 3-YSZ PH manufactured by Nippon Aerosil Co., Ltd.;
QSG-30 and QSG-80 manufactured by Shin-Etsu Chemical Co., Ltd.;
MSP-001, MSP-005, MSP-009, MSP-011, MSP-012, MSP-013, MSP-015, and MSP-016 manufactured by TAYCA Co., Ltd.; and
YA050C-SP3, YA050C, YA010C, and YC100C manufactured by Admatechs.

Preferred among these are Aerosil® NAX50 (fine silica particles, average primary particle diameter: 30 nm, methanol hydrophobicity: 28%, surface-treated with hexamethyldisilazane), QSG-30 (fine spherical silica particles, average primary particle diameter: 30 nm, methanol hydrophobicity: 67%, surface-treated with methyltrimethoxysilane and hexamethyldisilazane), MSP-011 (fine silica particles, average primary particle diameter: 30 nm, methanol hydrophobicity: 41%, surface-treated with methyltrimethoxysilane and hexamethyldisilazane), and YA050C-SP3 (fine spherical silica particles, average primary particle diameter: 50 nm, methanol hydrophobicity: 47%, surface-treated with phenylmethoxysilane).

The average primary particle diameter can be determined by a laser diffraction scattering method or by electron microscopy of particles. Specifically, a laser diffraction scattering method is more convenient for the measurement of particles 0.1 μm or larger, whereas electron microscopy is a more convenient method of particle diameter measurement for ultrafine particles of less than 0.1 μm. Here, 0.1 μm is a measured value by a laser diffraction scattering method. As

an example of a laser diffraction scattering method, the particle size may be measured by volume using a laser diffraction particle size distribution analyzer (SALD-2300 manufactured by Shimadzu Corporation) with a 0.2% sodium hexametaphosphate aqueous solution used as dispersion medium. A scanning electron microscope (e.g., SU3800 or S-4000 manufactured by Hitachi High-Technologies Corporation) may be used for electron microscopy. In electron microscopy, the particle size can be measured by taking an electron micrograph of particles, and the size of particles (at least 200 particles) observed in a unit field of the captured image may be measured using image-analyzing particle-size-distribution measurement software (Mac-View manufactured by Mountech Co., Ltd.). Here, the particle diameter is determined as an arithmetic mean value of the maximum and minimum lengths of particles, and the average primary particle diameter is calculated from the number of particles and the particle diameter.

Examples of the organic filler include polymethyl methacrylate, polyethyl methacrylate, a copolymer of polymethyl methacrylate and polyethyl methacrylate, a polymer of polyfunctional methacrylate, an ethylene-vinyl acetate copolymer, an acrylonitrile-butadiene-styrene copolymer, polyamides, polystyrene, polyvinyl chloride, chloroprene rubber, nitrile rubber, and styrene-butadiene rubber.

Examples of the inorganic/organic composite filler include those comprising an organic filler, and an inorganic filler dispersed in the organic filler, and those comprising an inorganic filler with various types of polymerizable monomers (A-2) coating the surface of the inorganic filler.

The filler (E) may be used alone, or two or more thereof may optionally be used in combination. In view of providing sufficient impact resistance, the content of filler (E) is preferably 20 mass% or less, more preferably 10 mass% or less, even more preferably 5 mass% or less in a total amount of the dental curable composition. In view of providing sufficient abrasion resistance and antifouling properties, the content of filler (E) is preferably 0.1 mass% or more, more preferably 0.5 mass% or more, even more preferably 1.0 mass% or more. The content of filler (E) may fall in any combinations of these ranges.

For example, the content of filler (E) is preferably 0.1 to 20 mass%, more preferably 0.5 to 10 mass%, even more preferably 1.0 to 5.0 mass%.

<Polysiloxane (F)>

The present invention may include a polysiloxane (F). The polysiloxane (F) is not particularly limited, as long as it is a polymer in which silicon atoms form bonds with each other through oxygen atoms, and at least some of the silicon atoms are linked to organic groups. The polysiloxane (F) improves the adhesive properties to the base material (preferably, a ceramic base material). The polysiloxane (F) may be used alone, or two or more thereof may be used in combination.

A certain preferred embodiment is, for example, a dental curable composition in which the polysiloxane (F) comprises a polysilsesquioxane (F-1).

The following descriptions of preferred embodiments are based on polysilsesquioxane (F-1).

<Polysilsesquioxane (F-1)>

The polysilsesquioxane (F-1) included in the present invention improves the adhesive properties to the base material (preferably, a ceramic base material). When combined with the filler (E), the polysilsesquioxane (F-1) can interact with the filler (E) to improve the affinity for the polymerizable compound (A), increasing the strength of the cured product.

The polysilsesquioxane (F-1) is not particularly limited, as long as it is a linear, branched, or network compound possessing Si-O bonds (siloxane bonds).

Examples of the polysilsesquioxane (F-1) include those with a constituent unit represented by the following general formula (2). In the following general formula (2), n = 1 in the case of polysilsesquioxane (F-1).

$$R_nSiO_{(4-n)/2} \qquad (2),$$

where R represents an organic group, and n represents a number exceeding 0 and less than 4.

Examples of R in the general formula (2) include an alkyl group, a cycloalkyl group, a haloalkyl group, an alkenyl group, an aryl group, and an arylalkyl group.

Examples of the alkyl group include C1 to C10 alkyl groups such as a methyl group, an ethyl group, a propyl group, and a butyl group.

Examples of the cycloalkyl group include C3 to C10 cycloalkyl groups such as a cyclopentyl group, and a cyclohexyl group.

Examples of the haloalkyl group include C1 to C10 halogenated alkyl groups such as a 3-chloropropyl group, and a 3,3,3-trifluoropropyl group.

Examples of the alkenyl group include C2 to C10 alkenyl groups such as a vinyl group, an allyl group, and a butenyl group.

Examples of the aryl group include C6 to C20 aryl groups such as a phenyl group, a tolyl group, and a naphthyl group.

Examples of the arylalkyl group include C1 to C4 alkyl groups with C6 to C12 aryl groups, such as a benzyl group, and a phenethyl group.

R is preferably a methyl group, a phenyl group, an alkyl group, an alkenyl group (such as a vinyl group), or a fluoro C1 to C6 alkyl group.

Examples of the polysilsesquioxane (F-1) include polydialkylsiloxanes such as polydimethylsiloxane; polyalkylalkenylsiloxanes such as polymethylvinylsiloxane; polyalkylarylsiloxanes such as polymethylphenylsiloxane; polydiarylsiloxanes such as polydiphenylsiloxane; and copolymers with the constituent polyorganosiloxane unit presented above, such as a dimethylsiloxane-methylvinylsiloxane copolymer, a dimethylsiloxane-methylphenylsiloxane copolymer, a dimethylsiloxane-methyl(3,3,3-trifluoropropyl)siloxane copolymer, and a dimethylsiloxane-methylvinylsiloxane-methylphenylsiloxane copolymer. The polysilsesquioxane (F-1) may be used alone, or two or more thereof may be used in combination.

The polysilsesquioxane (F-1) may use a composition prepared by heat treatment of an organosilicon compound such as a silane coupling agent. An example is a composition prepared by dissolving a silane coupling agent in a solvent, heating the solution at 70°C for 3 hours to obtain a sol solution, and removing the solvent and other components at 70°C by distillation in a vacuum. The polysilsesquioxane (F-1) is preferably one that is liquid at 25°C.

The polysilsesquioxane (F-1) obtained by such a method may be used in the form of a composition containing unreacted organosilicon compounds. In view of enhancing the curability of the dental curable composition, the polysilsesquioxane (F-1) may be used in the form of a composition containing unreactants, using organosilicon compounds having unsaturated groups (described later). By using a polysilsesquioxane (polysilsesquioxane composition) that is liquid at 25°C, it is easier to provide a uniform coating, improving the operability in building up the dental curable composition on the base material. A polysilsesquioxane composition that is liquid at 25°C can also reduce shade changes in coating the base material, providing even superior gloss retention.

Examples of the organosilicon compounds used include:

organosilicon compounds having a phenyl group, such as phenyltriethoxysilane, trimethoxy(phenylethyl)silane (a mixture of 1-phenylethyl and 2-phenylethyl), (1E,4E)-1-(4-hydroxy-3,5-dimethoxyphenyl)-5-(3,4,5-trimethoxyphenyl)-1,4-pentadien-3-one, trimethoxy(4-vinylphenyl)silane, trimethoxy(4-methoxyphenyl)silane, phenyltrimethoxysilane, and benzyltriethoxysilane;
organosilicon compounds having an alkyl group, such as methyltriethoxysilane, dodecyltrimethoxysilane, octadecyltriethoxysilane, n-octyltriethoxysilane, cyclohexyldimethoxymethylsilane, cyclohexyltrimethoxysilane, hexyltrimethoxysilane, ethyltrimethoxysilane, decyltrimethoxysilane, hexadecyltrimethoxysilane, propyltrimethoxysilane, cyclopentyltrimethoxysilane, butyltriethoxysilane, isooctyltriethoxysilane, and isobutyltriethoxysilane; and
organosilicon compounds having an unsaturated group, such as trimethoxy(4-vinylphenyl)silane, vinyltrimethoxysilane, 3-(trimethoxysilyl)propyl acrylate, 3-(triethoxysilyl)propyl methacrylate, allyltriethoxysilane, and allyltrimethoxysilane.

The organosilicon compounds may be used alone, or two or more thereof may be used as a mixture.

In view of improving the adhesive properties to ceramic base materials, and enhancing the affinity for the polymerizable compound (A) when combined with the filler (E) described below, preferred are organosilicon compounds having a phenyl group, organosilicon compounds having an unsaturated group, and mixtures of these, more preferably phenyltriethoxysilane, 3-(trimethoxysilyl)propyl acrylate, and mixtures of these.

In view of improving the adhesive properties to ceramic base materials, and enhancing the affinity for the polymerizable compound (A) when combined with the filler (E), the content of polysiloxane (F) (preferably, polysilsesquioxane (F-1)) is preferably 0.1 mass% or more, more preferably 0.2 mass% or more, even more preferably 0.5 mass% or more in a total amount of the dental curable composition.

In view of the hardness of the cured product, the content is preferably 10 mass% or less, more preferably 8 mass% or less, even more preferably 5 mass% or less. The range may be any combination of these upper and lower limits.

For example, the content of polysiloxane (F) is preferably 0.1 to 10 mass%, more preferably 0.2 to 8 mass%, even more preferably 0.5 to 5 mass%.

Optionally, the dental curable compositions used in the present invention may appropriately comprise additives such as solvents (e.g., organic solvents), colorants (pigments), ultraviolet absorbers, or antioxidants to such an extent that the addition of such additives does not hinder the effectiveness of the present invention.

In view of operability for easier uniform application to the base material, a dental curable composition of the present invention has a viscosity of preferably 10 to 1,000 mPa·s, more preferably 20 to 800 mPa·s, even more preferably

40 to 600 mPa·s. With these viscosity ranges, the curable composition can easily be coated over the desired area without causing runniness.

The viscosity of a dental curable composition of the present invention can be measured with, for example, a B-type viscometer (Brookfield viscometer) at 25°C and 20 rpm.

In view of abrasion resistance and impact resistance, a cured film (cured product) of a dental curable composition of the present invention has a pencil hardness of preferably F or higher, more preferably H or higher, even more preferably 2H or higher.

The pencil hardness of a cured film can be measured following JIS K 5600-5-4:1999, for example.

<Base Material>

Preferably, a dental curable composition of the present invention is used for applications as a coating agent for coating a base material. The following describes a base material coated in the present invention. The base material used may be selected from dental prosthesis materials that are commonly available. The base material may be used alone, or two or more thereof may be used in combination.

The base material preferably comprises at least one selected from the group consisting of zirconia, alumina, fused quartz, lithium silicate glass, an acrylic resin, and a composite material containing a polymer resin and an inorganic filler, more preferably zirconia. Here, "zirconia" comprises zirconia as the main component, and includes zirconia sintered bodies that result from sintering of zirconia.

Here, "main component" refers to the component that is most abundant, preferably 50 mass% or more, more preferably 60 mass% or more, even more preferably 80 mass% or more, particularly preferably 90 mass% or more.

Zirconia sintered bodies are primarily sintered crystal grains of zirconia. Accordingly, this specification is described through the case of zirconia that has not been sintered. However, in these descriptions, the term "zirconia" is interchangeable with "zirconia sintered body", and *vise versa,* provided that it does not pose any issue and unless otherwise specifically stated. For example, a zirconia sintered body may comprise zirconia, and a stabilizing agent capable of preventing a phase transformation of zirconia (hereinafter, also referred to simply as "stabilizing agent"). That is, a base material before sintering may comprise zirconia and a stabilizing agent.

Examples of the stabilizing agent include oxides such as calcium oxide (CaO), magnesium oxide (MgO), yttrium oxide ($Y_2O_3$), cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide (PreOn, $Pr_2O_3$), samarium oxide ($Sm_2O_3$), europium oxide ($Eu_2O_3$), and thulium oxide ($Tm_2O_3$). Preferred is yttria. The stabilizing agent may be used alone, or two or more thereof may be used in combination.

In the present invention, the content of the stabilizing agent in zirconia can be measured by, for example, inductively coupled plasma (ICP) emission spectral analysis or X-ray fluorescence analysis (XRF).

] In view of the strength and translucency of the sintered body, the yttria content of when the stabilizing agent contains yttria is preferably 3.0 to 7.5 mol%, more preferably 3.5 to 7.0 mol%, even more preferably 4.0 to 6.5 mol% relative to the total mole of zirconia and stabilizing agent. The sintered body can increase its translucency with a yttria content of 3.0 mol% or more, whereas a decrease in the strength of the sintered body can be reduced with a yttria content of 7.5 mol% or less.

When the zirconia contains calcium oxide, the content of calcium oxide is preferably 1 mol% or less, more preferably 0.3 mol% or less in total 100 mol% of zirconia and stabilizing agent.

When the zirconia contains magnesium oxide, the content of magnesium oxide is preferably 1 mol% or less, more preferably 0.3 mol% or less in total 100 mol% of zirconia and stabilizing agent.

When the zirconia contains cerium oxide, the content of cerium oxide is preferably 1 mol% or less, more preferably 0.3 mol% or less in total 100 mol% of zirconia and stabilizing agent.

Aside from a sintered body obtained by sintering molded zirconia particles under ordinary pressure or under no applied pressure, the zirconia sintered body includes sintered bodies compacted by a high-temperature pressing process such as HIP (Hot Isostatic Pressing).

Preferably, the zirconia sintered body has at least one of partially stabilized zirconia and fully stabilized zirconia as the matrix phase. In the zirconia sintered body, the primary crystalline phase of zirconia is at least one of the tetragonal crystal system and cubical crystal system. The zirconia may comprise both tetragonal crystal system and cubical crystal system. Preferably, the zirconia sintered body is essentially free of a monoclinic crystal system. Zirconia that is partially stabilized by adding a stabilizing agent is called partially stabilized zirconia (PSZ), whereas the term fully stabilized zirconia is used to refer to zirconia that is fully stabilized.

The shape and size (dimensions) of the base material can be appropriately selected according to factors such as use, and the oral environment of the patient.

The following describes a dental prosthesis of the present invention.

A dental prosthesis of the present invention is a dental prosthesis comprising a base material and a resin layer,

and the resin layer comprises a polymerized and cured product of a curable composition comprising the polymerizable compound (A), polyfunctional thiol (B-1), fluorescent agent (C), and polymerization initiator (D). The base material is coated with the resin layer, partly or completely. The resin layer is obtained by applying the dental curable composition onto the base material, followed by polymerization and cure.

A dental prosthesis of the present invention may comprise two or more resin layers of different compositions. Two or more resin layers may be layered on the base material.

The translucency improves when the surface of the base material is coated with the resin layer, compared with the base material alone. When excessively thin, the resin layer fails to sufficiently exhibit the translucency improving effect, whereas the translucency tends to decrease when the resin layer is excessively thick. Accordingly, the thickness of the resin layer on the base material (the total thickness when two or more resin layers of different compositions are present) is preferably 1 to 500 μm, more preferably 7 to 100 μm, even more preferably 7 to 80 μm, most preferably 7 to 50 μm. Preferably, the resin layer thickness is the thickness at the thickest portion of the resin layer.

A method of production of the dental prosthesis is described below.

First, a base material of a predetermined shape and dimensions is prepared.

For example, the base material is fabricated into a dental prosthesis for the patient, using a known method. This is followed by application of the dental curable composition to the base material. Optionally, an adhesive composition (G), separately prepared in advance, is applied to the area of base material where the dental curable composition is applied. A brush can be used for application, for example. The area of base material where the dental curable composition is applied may be appropriately selected according to the oral environment of the patient. Preferably, the dental curable composition is applied to an area that will be exposed inside the oral cavity. The dental curable composition may be applied to an area facing the abutment tooth.

The dental curable composition together with the base material is polymerized after the dental curable composition was applied to the base material. For example, polymerization can take place for 90 seconds with an irradiator that emits light of the 500 nm wavelength range. The same process may be repeated when forming a plurality of resin layers as a polymerized and cured product of the dental curable composition. A dental prosthesis of the present invention can then be obtained after optional shape modification and polishing.

In another preferred method, an adhesive composition (G) comprising an adhesive monomer (a-4) having an acidic group may bond the interface between the base material and the dental curable composition.

From the perspective of both ease of application and adhesive properties, another preferred method applies the dental curable composition after a primer composition (G) comprising an adhesive monomer (a-4) having an acidic group and a volatile organic solvent (H) is applied to the base material in advance.

As an example, the volatile organic solvent (H) contained in the primer composition (G) is preferably one having a boiling point of preferably 150°C or less, more preferably 100°C or less at ordinary pressure.

Using a volatile organic solvent having a boiling point exceeding 150°C at ordinary pressure may result in a decrease in the surface curability of the dental curable composition.

Examples of the volatile organic solvent (H) include alcohols such as ethanol, methanol, 1-propanol, and isopropyl alcohol; ketones such as acetone, methyl ethyl ketone, and diethyl ketone; ethers such as 1,2-dimethoxyethane, 1,2-diethoxyethane, and tetrahydrofuran; esters such as methyl formate, ethyl formate, methyl acetate, ethyl acetate, propyl acetate, and butyl acetate; and (meth)acrylic acid esters such as methyl (meth)acrylate, ethyl (meth)acrylate, n-propyl (meth)acrylate, and isopropyl (meth)acrylate. In view of the ability to cure simultaneously with the polymerizable monomers, (meth)acrylic acid esters are preferred. In view of even lower toxicity and lower boiling point, methyl methacrylate is particularly preferred.

The volatile organic solvent (H) may be used alone, or two or more thereof may be used in combination. In view of coatability (operability), the content of volatile organic solvent (H) is preferably 90 to 99.9 mass%, more preferably 95 to 99 mass%, even more preferably 97 to 99 mass% in a total amount of primer composition (G).

A dental prosthesis of the present invention can achieve higher transparency than that achievable with the base material alone, imparting fluorescence. A dental prosthesis of the present invention can also maintain a higher level of glossiness and fluorescence compared to the base material on its own. In this way, a dental prosthesis of the present invention can present an appearance that more closely resemble natural teeth than when the base material is used alone.

The present invention encompasses embodiments combining the foregoing features in various ways within the technical idea of the present invention, provided that the present invention can exhibit its effects.

## EXAMPLES

The present invention is described below in greater detail by way of Examples. However, the present invention is in no way limited by the following descriptions, and various changes may be made by a person with ordinary knowledge in the art within the technical idea of the present invention.

[Urethane (Meth)Acrylate Oligomer (A-1)]

UV1700B: urethane acrylate oligomer (UV1700B manufactured by Mitsubishi Chemical Corporation under this trade name; viscosity at 25°C: 40,000 to 100,000 mPa s; pencil hardness of cured film: 4H)
UA-306T pentaerythritol triacrylate toluene diisocyanate urethane prepolymer (UA-306T manufactured by Kyoeisha Chemical Co., Ltd. under this trade name)

[Polymerizable Monomer (A-2)]

SR454NS: ethoxylated (3)trimethylolpropane triacrylate (SR454NS manufactured by Sartomer Company, Exton, PA)
SR9003NS: propoxylated (2)neopentyl glycol diacrylate (SR9003NS manufactured by Sartomer Company, Exton, PA)
DPHA: dipentaerythritol hexaacrylate (DPHA manufactured by Sartomer Company, Exton, PA)
U4TH: N,N-(2,2,4-trimethylhexamethylene)bis[2-(aminocarboxy)propane-1,3-diol]tetramethacryl ate

[Polyfunctional Thiol (B-1)]

MT-PE-1: pentaerythritol tetrakis(3-mercaptobutyrate) (KarenzMT® PE1 manufactured by Showa Denko K.K. under this trade name)
3-MPO: 3-(3-mercapto-propoxy)-2,2-bis-(3-mercapto-propoxymethyl)-propan-1-ol

[Fluorescent Agent (C)]

[Inorganic Fluorescent Agent (C-1)]

$Y_2SiO_5$:Ce

[Organic Fluorescent Agent (C-2)]

DHTP: diethyl 2,5-dihydroxyterephthalate

[Polymerization Initiator (D)]

BAPO: bis(2,4,6-trimethylbenzoyl)phenylphosphine oxide
Lucirin-TPO: 2,4,6-trimethylbenzoyldiphenylphosphine oxide
CQ: dl-camphorquinone

[Filler (E)]

QSG-30: spherical fine silica particles manufactured by Shin-Etsu Chemical Co., Ltd.; average primary particle diameter: 30 nm; methanol hydrophobicity: 67%; surface-treated with methyltrimethoxysilane and hexamethyldisilazane
R7200: AEROSIL® R7200 manufactured by EVONIK INDUSTRIES under this trade name (average primary particle diameter: 12 nm; hydrophobic fumed silica; surface-treated with a methacryloyloxysilyl group-containing silane compound)

[Others]

DMBE: ethyl 4-(N,N-dimethylamino)benzoate

[Polysiloxane (F)]

A sol solution of a polysilsesquioxane composition was prepared by mixing 5.9 g (24.5 mmol) of phenyltriethoxysilane, 51.1 g (218.1 mmol) of 3-(trimethoxysilyl)propyl acrylate, 23.4 g (1.3 mol) of water, 9.2 g (0.2 mol) of ethanol, and 0.3 g (4.2 mmol) of acetic acid, and heating the mixture at 70°C for 3 hours. Subsequently, the solvent was distilled away by applying heat at 70°C for 10 minutes in a vacuum, yielding a polysilsesquioxane composition (F-1) that is liquid at 25°C.

[Monofunctional Thiol]

3-MPE: ethyl 3-mercaptopropionate

Examples 1 to 34 and Comparative Examples 1 to 7

Dental curable compositions and base materials were prepared for Examples and Comparative Examples as follows, and their properties were evaluated. The results are presented in Tables 1 to 4.

[Preparation of Dental Curable Composition]

To prepare the dental curable compositions, the components shown in Tables 1 to 4 were mixed at ordinary temperature in the mass ratios shown in Tables 1 to 4, using a planetary mixer (Mazerustar, manufactured by Kurabo Ltd.).

[Preparation of Zirconia Ceramic Material as Base Material]

To prepare samples corresponding to dental prostheses of the present invention, a dental zirconia (KATANA® Zirconia STML NW manufactured by Kuraray Noritake Dental Inc. under this trade name; thickness: 14 mm) was milled into a hemispherical shape (12 mm in diameter), a cuboidal shape (2.4 mm in thickness, 36 mm in length, 24 mm in width), or a disc shape (22 mm in diameter, 1.2 mm in thickness), using a dental milling machine DWX-51D (manufactured by Roland DG Corporation under this trade name).

Subsequently, the shaped product was fired at 1,550°C for 2 hours with a furnace (Noritake KATANA® F-1N manufactured by SK Medical Electronics Co., Ltd. under this trade name), yielding a base material formed of a zirconia sintered body of a hemispherical shape (10 mm in diameter), a cuboidal shape (2 mm in thickness, 30 mm in length, 20 mm in width), or a disc shape (18 mm in diameter, 1 mm in thickness). The hemisphere surface or disc surface was then sandblasted with 50 μm alumina particles under 0.2 MPa pressure, and the base material was ultrasonically washed in acetone, and dried to prepare a cuboidal or disc-shaped zirconia base material in preparation for coating.

[Viscosity Measurement of Dental Curable Composition]

The dental curable compositions prepared earlier were measured with a B-type viscometer (Brookfield viscometer) under 25°C, 20 rpm conditions, and mean values were calculated (n = 3).

[Evaluation of Operability of Dental Curable Composition]

The dental curable compositions prepared earlier were evaluated for the operability of application on the zirconia base materials prepared, using the following criteria (n = 5).

A: Formation of a uniform thin film is possible by single application
B: streaks or lines occur after single application, but formation of a uniform thin film is possible after multiple applications
C: Formation of a uniform coating surface is not possible even after multiple applications

For n = 5, the composition was categorized as A when three or more of its samples received an A score, and categorized as C when three or more of its samples received a C score. A composition was labeled as B when neither condition was met by the samples.

The evaluations of various properties below were conducted using polymerized and cured products of the dental curable compositions prepared earlier, after curing the compositions under light using the curing conditions (seconds) presented in Tables 1 to 4. The measurement of pencil hardness was conducted on the polymerized and cured dental curable compositions (without application to the base material). For the evaluation of fluorescence, the dental curable composition was applied to the base material and cured as in the evaluation of abrasion resistance and gloss retention.

[Measurement of Pencil Hardness of Polymerized and Cured Dental Curable Composition]

Measurement was conducted on cured products of the dental curable compositions prepared earlier, following JIS K 5600-5-4:1999 (n = 1).

[Abrasion Resistance and Gloss Retention Evaluation for Polymerized and Cured Dental Curable Composition: Abrasion Test]

A Clearfil® Ceramic® Primer Plus (manufactured by Kuraray Noritake Dental Inc.) was applied to the cuboidal zirconia base material prepared earlier. After drying the material by blowing air, the dental curable composition prepared earlier was applied, and cured under light using the curing conditions of Tables 1 to 4 to yield a specimen with the polymerized and cured product coating the surface of the base material. For photoirradiation, a dental laboratory LED polymerizer (Alpha Light® II manufactured by J. Morita Corp.) was used.
With a commercially available toothbrush (Between, Zigzag Bristles, Regular Cut; manufactured by Lion Corporation) secured to a stroke-type abrasion tester (manufactured by TOKYO GIKEN INC.), an abrasion test was conducted under a 250 g load in 40,000 cycles with the coating surface of the specimen facing upward, in the presence of a suspension of a commercially available tooth paste (Dentor Clear MAX (Lion)/distilled water = 90/10 parts by mass).
After the abrasion test, the glossiness of the specimen surface was measured with a gloss meter (VG-107 manufactured by Nippon Denshoku Industries Co., Ltd.) (n = 2), and the mean value was determined as a fraction relative to the glossiness of a mirror at 100%. The measurement was conducted at 60° angle.
In view of the gloss retention to maintain the same level of gloss as natural teeth over extended time periods, the preferred glossiness is 70% or more, more preferably 75% or more, even more preferably 80% or more, particularly preferably 85% or more.
Separately, surface roughness (arithmetic mean roughness Ra) was measured with a surface roughness meter Laser Focus Displacement Gauge LT-8100 (manufactured by Keyence) after the abrasion test, and mean values were calculated (n = 2).
In view of superior abrasion resistance, the preferred arithmetic mean roughness Ra is 3.8 μm or less, more preferably 3.3 μm or less, even more preferably 2.8 μm or less, particularly preferably 2.3 μm or less.
The arithmetic mean roughness Ra may be, for example, 1.0 μm or more. In the table, "Surface roughness after wear" represents arithmetic mean roughness Ra.

[Fluorescence Evaluation for Polymerized and Cured Dental Curable Composition]

The dental curable composition was applied to the base material in the same manner as in the evaluation of abrasion resistance and gloss retention. Subsequently, a cured product of the dental curable composition prepared earlier was subjected to fluorescence spectrum measurement using a fluorescence spectrophotometer (F-7100 manufactured by Hitachi High-Tech Science Corporation) in fluorescence mode with an excitation wavelength set at 366 nm. The average value was calculated for n = 3. Rhodamine B was used to correct spectra across different instruments. Within the spectrum obtained, the fluorescence wavelength displaying the highest intensity in the fluorescence wavelength range of 430 to 500 nm was identified as the maximum wavelength, and the intensity at this maximum wavelength was determined as the fluorescence intensity.
In view of providing fluorescence comparable to the fluorescence of natural teeth, the preferred fluorescence intensity is 120 to 6,000, more preferably 350 to 5,800, even more preferably 550 to 5,500.

[Fluorescence Durability Evaluation for Polymerized and Cured Dental Curable Composition]

The fluorescence after the abrasion test was evaluated using the method described earlier. Specifically, the retention of fluorescence intensity was calculated as a measure of the percentage change in fluorescence intensity before and after the abrasion test.

Retention of fluorescence intensity (%) = (fluorescence intensity after abrasion test / fluorescence intensity before abrasion test) × 100

In the formula, the fluorescence intensity before abrasion test represents the fluorescence intensity calculated in the fluorescence evaluation for polymerized and cured dental curable composition, whereas the fluorescence intensity after abrasion test represents the fluorescence intensity calculated for specimens after abrasion test using the method described in the fluorescence evaluation for polymerized and cured dental curable composition.

In view of fluorescence durability that allows for the maintenance of fluorescence comparable to that of natural teeth over extended time periods, the preferred retention of fluorescence intensity is 80% or more, more preferably 85% or more, even more preferably 90% or more, particularly preferably 95% or more.

[Measurement of Shade Change in Base Material]

The disc-shaped zirconia base material prepared earlier was measured for chromaticity according to the L*a*b* evaluation system (JIS Z 8781-4:2013 Color Measurements - Part 4: CIE 1976 L*a*b* color space), using a spectrophotometer Crystaleye (manufactured by Olympus Corporation under this trade name) in 7-band measurement mode with an LED light source, yielding L*0, a*0, and b*0. Subsequently, a Clearfil® Ceramic® Primer Plus (manufactured by Kuraray Noritake Dental Inc.) was applied onto the disc-shaped zirconia base material prepared earlier. After drying the material by blowing air, the dental curable composition prepared earlier was applied, and cured into a specimen under the curing conditions shown in Tables 1 to 4.

The specimen was measured for chromaticity using the same method used for the chromaticity measurement of the zirconia base material, yielding L*1, a*1, and b*1. The measured values were substituted in the formula below to determine ΔE* as an index of color change (n = 1).

$$\Delta E^* = \{(L^*1 - L^*0)^2 + (a^*1 - a^*0)^2 + (b^*1 - b^*0)^2\}^{1/2}$$

In view of the effect on the shade of the base material, the color difference ΔE* should preferably take values as small as possible.

The preferred value of color difference ΔE* is 1.6 or less, more preferably 1.4 or less, even more preferably 1.2 or less, most preferably 1.0 or less.

[Table 1]

| Components (parts by mass) | Compound | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|
| Urethane (meth)acrylate oligomer (A-1) | UV1700B | 36.0 | 36.0 | 36.0 | 36.0 | 36.0 | 36.0 | 37.0 | 36.0 | 36.0 | 36.0 | 33.0 | 17.0 | 17.0 |
| | UA-306T | | | | | | | | | | | | 19.0 | 19.0 |
| Polymerizable monomer (A-2) | SR454NS | 41.9 | 41.5 | 41.0 | 40.9 | 40.5 | 40.0 | 31.5 | 40.9 | 40.9 | 36.0 | 36.0 | 2.0 | 2.0 |
| | SR9003NS | | | | | | | | | | | | 38.4 | 38.4 |
| | DPHA | | | | | | | | | | | | | |
| | U4TH | | | | | | | | | | | | | |
| Polyfunctional thiol (B-1) | MT-PE-1 | 19.5 | 19.5 | 19.5 | 19.5 | 19.5 | 19.5 | 26.5 | 19.5 | 19.5 | 19.5 | 19.5 | 20.0 | 20.0 |
| | 3-MPO | | | | | | | | | | | | | |
| Inorganic fluorescent agent (C-1) | Y2SiO5:Ce | | | | | | | | | | 5.0 | 8.0 | | |
| Organic fluorescent agent (C-2) | DHTP | 0.1 | 0.5 | 0.05 | 0.1 | 0.5 | 1.0 | 1.0 | 0.1 | 0.1 | | | 0.1 | 0.1 |
| Filler (E) | QSG-30 | 2.5 | 2.5 | 2.5 | 2.5 | 2.5 | 2.5 | 2.5 | 2.5 | 2.5 | 2.5 | 2.5 | 2.5 | 2.5 |
| | R7200 | | | | | | | | | | | | | |
| Polysiloxane (F) | Composition F-1 | | | 1.0 | 1.0 | 1.0 | 1.0 | 1.5 | 1.0 | 1.0 | 1.0 | 1.0 | 1.0 | 1.0 |
| Monofunctional thiol | 3-MPE | | | | | | | | | | | | | |
| Polymerization initiator (D) | BAPO | 4.0 | 4.0 | 4.0 | 4.0 | 4.0 | 4.0 | 4.0 | 4.0 | 4.0 | 4.0 | 4.0 | 4.0 | |
| | Lucirin-TPO | | | | | | | | | | | | | 5.0 |
| | CQ | | | | | | | | | | | | | |
| Others | DMBE | | | | | | | | | | | | | |
| Composition viscosity (mPa·s) | | 80 | 80 | 100 | 100 | 100 | 100 | 500 | 100 | 100 | 100 | 100 | 100 | 100 |
| Composition operability | | A | A | A | A | A | A | A | A | A | A | A | A | A |
| Pencil hardness of cured film | | 4H | 4H | 4H | 4H | 4H | 4H | 4H | 4H | 4H | 4H | 4H | 4H | 4H |
| Curing conditions (photoirradiation time (sec)) | | 10s | 10s | 10s | 10s | 10s | 10s | 10s | 20 s | 30 s | 10s | 10s | 10s | 10s |
| Fluorescence of cured product | Fluorescence intensity (1) | 1677 | 3879 | 940 | 1675 | 3876 | 4972 | 5095 | 1772 | 1829 | 159 | 281 | 1861 | 1872 |
| | Maximum wavelength (nm) | 448 | 448 | 448 | 447 | 448 | 447 | 445 | 447 | 447 | 444 | 443 | 448 | 448 |

(continued)

| Components (parts by mass) | | Compound | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 |
|---|---|---|---|---|---|---|---|---|---|---|---|---|---|---|---|
| Abrasion test | Glossiness after wear (%) | | 85.3 | 84.5 | 87.8 | 87.6 | 87.2 | 86.9 | 86.9 | 88.0 | 86.7 | 87.1 | 86.1 | 88.8 | 89.1 |
| | Surface roughness after wear (μm) | | 1.98 | 2.03 | 1.75 | 1.76 | 1.78 | 1.79 | 1.75 | 1.66 | 1.89 | 1.81 | 1.88 | 1.63 | 1.60 |
| | Fluorescence after wear | Fluorescence intensity (2) | 1594 | 3721 | 923 | 1598 | 3678 | 4769 | 4891 | 1689 | 1799 | 157 | 279 | 1797 | 1826 |
| | | Retention of fluorescence intensity ((2)/(1) (%)) | 95 | 96 | 98 | 95 | 95 | 96 | 96 | 95 | 98 | 99 | 99 | 97 | 98 |
| Evaluation of shade change ($\Delta E^*$) | | | 0.65 | 0.57 | 0.78 | 0.81 | 0.83 | 0.85 | 0.77 | 0.35 | 0.72 | 0.88 | 0.91 | 0.26 | 0.20 |

[Table 2]

| Components (parts by mass) | Compound | Ex. 14 | Ex. 15 | Ex. 16 | Ex. 17 | Ex. 18 | Ex. 19 | Ex. 20 | Ex. 21 | Ex. 22 | Ex. 23 | Ex. 24 | Ex. 25 | Ex. 26 |
|---|---|---|---|---|---|---|---|---|---|---|---|---|---|---|
| Urethane (meth)acrylate oligomer (A-1) | UV1700B | 17.0 | 17.0 | 17.0 | 17.0 | 17.0 | 17.0 | 17.0 | 17.0 | 40.0 | 52.9 | 64.9 | 17.0 | 36.0 |
| | UA-306T | 19.0 | 19.0 | 19.0 | 19.0 | 19.0 | 19.0 | 19.0 | 19.0 | | | | 19.0 | |
| Polymerizable monomer (A-2) | SR454NS | 2.0 | 2.0 | 2.0 | 2.0 | 2.0 | 2.0 | 2.0 | 2.0 | 43.6 | 30.7 | 18.7 | | 36.0 |
| | SR9003NS | 38.4 | 18.5 | 39.4 | 39.4 | 40.9 | 33.0 | 41.9 | 34.0 | | | | | |
| | DPHA | | 199 | | | | | | | | | | | |
| | U4TH | | | | | | | | | | | | 40.4 | |
| Polyfunctional thiol (B-1) | MT-PE-1 | | | 20.0 | 20.0 | 20.0 | 20.0 | 20.0 | 20.0 | 12.8 | 12.8 | 12.8 | 20.0 | 19.5 |
| | 3-MPO | 20.0 | 20.0 | | | | | | | | | | | |
| Inorganic fluorescent agent (C-1) | Y2SiO5:Ce | | | | | | 8.0 | | 8.0 | | | | | |
| Organic fluorescent agent (C-2) | DHTP | 0.1 | 0.1 | 0.1 | 0.1 | 0.1 | | 0.1 | | 0.1 | 0.1 | 0.1 | 0.1 | 5.0 |
| Filler (E) | QSG-30 | 2.5 | 2.5 | 2.5 | 2.5 | | | | | | 2.5 | 2.5 | 2.5 | 2.5 |
| | R7200 | | | | | | | | | 2.5 | | | | |
| Polysiloxane (F) | Composition F-1 | 1.0 | 1.0 | | | 1.0 | 1.0 | | | 1.0 | 1.0 | 1.0 | 1.0 | 1.0 |
| Monofunctional thiol | 3-MPE | | | | | | | | | | | | | |
| Polymerization initiator (D) | BAPO | 4.0 | 1.4 | 4.0 | | | | | | 4.0 | 4.0 | 4.0 | | 4.0 |
| | Lucirin-TPO | | | | 5.0 | 5.0 | 5.0 | 5.0 | 5.0 | | | | 5.0 | |
| | CQ | | 1.4 | | | | | | | | | | | |
| Others | DMBE | | 12 | | | | | | | | | | | |
| Composition viscosity (mPa s) | | 100 | 500 | 80 | 80 | 40 | 40 | 30 | 30 | 100 | 100 | 100 | 100 | 100 |
| Composition operability | | A | A | A | A | A | A | A | A | A | A | A | A | A |
| Pencil hardness of cured film | | 4H | 4H | 4H | 4H | 4H | 4H | 4H | 4H | 4H | 4H | 4H | 4H | 4H |
| Curing conditions (photoirradiation time (sec)) | | 10s | 10s | 10s | 10s | 10s | 10s | 10s | 10s | 10s | 10s | 10s | 10s | 10s |
| Fluorescence of cured product | Fluorescence intensity (1) | 1758 | 1678 | 1811 | 1823 | 1859 | 302 | 1863 | 304 | 1799 | 1791 | 1801 | 1852 | 6048 |
| | Maximum wavelength (nm) | 448 | 448 | 448 | 448 | 447 | 444 | 447 | 444 | 447 | 447 | 447 | 448 | 447 |

(continued)

| Components (parts by mass) | | Compound | Ex. 14 | Ex. 15 | Ex. 16 | Ex. 17 | Ex. 18 | Ex. 19 | Ex. 20 | Ex. 21 | Ex. 22 | Ex. 23 | Ex. 24 | Ex. 25 | Ex. 26 |
|---|---|---|---|---|---|---|---|---|---|---|---|---|---|---|---|
| Abrasion test | Glossiness after wear (%) | | 75.4 | 82.2 | 85.8 | 86.1 | 75.4 | 80.1 | 72.4 | 76.8 | 84.8 | 86.3 | 86.8 | 88.9 | 86.0 |
| | Surface roughness after wear (μm) | | 3.12 | 2.51 | 1.95 | 1.91 | 2.96 | 2.78 | 3.02 | 2.95 | 2.28 | 2.01 | 1.85 | 1.62 | 1.96 |
| | Fluorescence after wear | Fluorescence intensity (2) | 1509 | 1567 | 1720 | 1730 | 1597 | 271 | 1527 | 262 | 1683 | 1702 | 1733 | 1802 | 5788 |
| | | Retention of fluorescence intensity ((2)/(1) (%)) | 86 | 93 | 95 | 95 | 86 | 90 | 82 | 86 | 94 | 95 | 96 | 97 | 96 |
| Evaluation of shade change (ΔE*) | | | 0.80 | 1.45 | 0.24 | 0.21 | 0.20 | 0.89 | 0.19 | 0.81 | 0.48 | 0.41 | 0.43 | 0.20 | 0.99 |

[Table 3]

| Components (parts by mass) | | | Compound | Ex. 27 | Ex. 28 | Ex. 29 | Ex. 30 | Ex. 31 | Ex. 32 | Ex. 33 | Ex. 34 |
|---|---|---|---|---|---|---|---|---|---|---|---|
| Urethane (meth)acrylate oligomer (A-1) | | | UV1700B | 26.3 | 31.8 | 27.3 | 24.9 | 26.1 | 21.3 | 21.0 | 23.0 |
| | | | UA-306T | 31.0 | 37.5 | 32.9 | 29.5 | 30.9 | 25.2 | 24.0 | 25.0 |
| Polymerizable monomer (A-2) | | | SR454NS | 1.5 | 0.9 | 1.5 | 1.5 | 1.5 | 1.4 | 2.0 | 2.0 |
| | | | SR9003NS | 27.7 | 16.3 | 28.3 | 26.2 | 27.5 | 22.7 | 43.5 | 44.5 |
| | | | DPHA | | | | | | | | |
| | | | U4TH | | | | | | | | |
| Polyfunctional thiol (B-1) | | | MT-PE-1 | 9.9 | 9.9 | 9.9 | 9.9 | 9.9 | 9.9 | 5.0 | 1.0 |
| | | | 3-MPO | | | | | | | | |
| Inorganic fluorescent agent (C-1) | | | Y2SiO5:Ce | | | | | | | | |
| Organic fluorescent agent (C-2) | | | DHTP | 0.1 | 0.1 | 0.1 | 3.0 | 0.1 | 6.0 | 1.0 | 1.0 |
| Filler (E) | | | QSG-30 | 2.5 | 2.5 | | 5.0 | | 12.5 | 2.5 | 2.5 |
| | | | R7200 | | | | | | | | |
| Polysiloxane (F) | | | Composition F-1 | 1.0 | 1.0 | | | 4.0 | 1.0 | 1.0 | 1.0 |
| Monofunctional thiol | | | 3-MPE | | | | | | | | |
| Polymerization initiator (D) | | | BAPO | | | | | | | | |
| | | | Lucirin-TPO | 5.0 | 5.0 | 5.0 | 5.0 | 5.0 | 5.0 | 5.0 | 5.0 |
| | | | CQ | | | | | | | | |
| Others | | | DMBE | | | | | | | | |
| Composition viscosity (mPa·s) | | | | 100 | 100 | 100 | 250 | 100 | 1000 | 500 | 700 |
| Composition operability | | | | A | A | A | A | A | A | A | A |
| Pencil hardness of cured film | | | | 4H | 4H | 4H | 4H | 4H | 4H | 4H | 4H |
| Curing conditions (photoirradiation time (sec)) | | | | 10 s | 10 s | 10 s | 10 s | 10 s | 10 s | 10 s | 10 s |
| Fluorescence of cured product | Fluorescence intensity (1) | | | 1789 | 1758 | 1751 | 5221 | 1745 | 5921 | 4821 | 4757 |
| | Maximum wavelength (nm) | | | 448 | 448 | 447 | 448 | 447 | 449 | 448 | 448 |
| Abrasion test | Glossiness after wear (%) | | | 83.8 | 84.2 | 72.4 | 84.8 | 75.4 | 84.1 | 82.1 | 80.2 |
| | Surface roughness after wear (μm) | | | 2.08 | 2.22 | 3.02 | 2.43 | 2.96 | 1.55 | 2.01 | 2.25 |
| | Fluorescence after wear | Fluorescence intensity (2) | | 1638 | 1645 | 1402 | 4877 | 1431 | 5621 | 4462 | 4134 |
| | | Retention of fluorescence intensity ((2)/(1) (%)) | | 92 | 94 | 80 | 93 | 82 | 95 | 93 | 87 |
| Evaluation of shade change (ΔE*) | | | | 0.32 | 0.33 | 0.33 | 0.36 | 0.34 | 0.43 | 0.89 | 1.00 |

[Table 4]

| Components (parts by mass) | | | Compound | Com. Ex. 1 | Com. Ex. 2 | Com. Ex. 3 | Com. Ex. 4 | Com. Ex. 5 | Com. Ex. 6 | Com. Ex. 7 |
|---|---|---|---|---|---|---|---|---|---|---|
| Urethane (meth)acrylate oligomer (A-1) | | | UV1700B | 36.0 | 45.5 | 45.5 | 45.5 | 36.0 | 36.0 | |
| Polymerizable monomer (A-2) | | | SR454NS | 42.0 | 50.9 | 50.5 | 46.0 | 40.9 | 36.0 | |
| Polyfunctional thiol (B-1) | | | MT-PE-1 | 19.5 | | | | | | 91.5 |
| Inorganic fluorescent agent (C-1) | | | Y2SiO5:Ce | | | | 5.0 | | 5.0 | 5.0 |
| Organic fluorescent agent (C-2) | | | DHTP | | 0.1 | 0.5 | | 0.1 | | |
| Filler (E) | | | QSG-30 | 2.5 | 2.5 | 2.5 | 2.5 | 2.5 | 2.5 | 2.5 |
| Polysiloxane (F) | | | Composition F-1 | | 1.0 | 1.0 | 1.0 | 1.0 | 1.0 | 1.0 |
| Monofunctional thiol | | | 3-MPE | | | | | 19.5 | 19.5 | |
| Polymerization initiator (D) | | | BAPO | 4.0 | 4.0 | 4.0 | 4.0 | 4.0 | 4.0 | 4.0 |
| Composition viscosity (mPa·s) | | | | 80 | | | | | | |
| Composition operability | | | | A | B | B | B | A | A | |
| Pencil hardness of cured film | | | | 4H | | | | | | Failed evaluation due to inadequate curing |
| Curing conditions (photoirradiation time (sec)) | | | | 10s | 20 s | 20 s | 20 s | 20 s | 20 s | |
| Fluorescence of cured product | Fluorescence intensity (1) | | | 0 | 1249 | 3776 | 105 | 1421 | 119 | |
| | Maximum wavelength (nm) | | | | 445 | 444 | 444 | 445 | 444 | |
| Abrasion test | Glossiness after wear (%) | | | 82.1 | 39.2 | 37.2 | 34.4 | 46.8 | 67.2 | |
| | Surface roughness after wear (μm) | | | 1.98 | 10.01 | 11.74 | 10.32 | 6.91 | 3.57 | |
| | Fluorescence after wear | Fluorescence intensity (2) | | 0 | 591 | 1521 | 52 | 987 | 76 | |
| | | Retention of fluorescence intensity ((2)/(1) (%)) | | | 47 | 40 | 50 | 69 | 64 | |
| Evaluation of shade change (ΔE*) | | | | 0.65 | 6.08 | 6.56 | 6.77 | 7.23 | 6.11 | |

As shown in Tables 1 to 4, Examples 1 to 34 were superior to Comparative Examples 2 to 7 in terms of the gloss retention and fluorescence durability of the cured product, and the effectiveness of shade change reduction in the experiments simulating the oral environment.

Examples 1 to 34 were superior to Comparative Examples 2 to 4 in terms of operability.

Examples 1 to 34 were superior to Comparative Examples 2 to 5 in terms of the abrasion resistance of the cured product.

Examples 1 to 34 were superior to Comparative Examples 4 and 6 in terms of fluorescence. Comparative Example 1, lacking the fluorescent agent (C), did not show fluorescence.

Comparative Example 7, lacking the polymerizable compound (A), failed to sufficiently cure, preventing evaluations of properties.

## Claims

1. A dental curable composition comprising a polymerizable compound (A), a polymerization accelerator (B), a fluorescent agent (C), and a polymerization initiator (D),
   wherein the polymerization accelerator (B) comprises a polyfunctional thiol (B-1) having two or more mercapto groups per molecule.

2. The dental curable composition according to claim 1, wherein the polyfunctional thiol (B-1) comprises a polyfunctional thiol having 2 to 4 mercapto groups per molecule.

3. The dental curable composition according to claim 1 or 2, wherein the mercapto groups in the polyfunctional thiol (B-1) are bound to a secondary carbon atom or a tertiary carbon atom.

4. The dental curable composition according to any one of claims 1 to 3, wherein the fluorescent agent (C) comprises an organic fluorescent agent (C-2).

5. The dental curable composition according to claim 4, wherein the content of the organic fluorescent agent (C-2) is 0.001 to 10 mass%.

6. The dental curable composition according to any one of claims 1 to 5, wherein the mass ratio (B-1):(C) of the content of the polyfunctional thiol (B-1) and the content of the fluorescent agent (C) is 100:0.001 to 100:600.

7. The dental curable composition according to any one of claims 1 to 6, wherein the polymerizable compound (A) comprises a urethane (meth)acrylate oligomer (A-1) and/or a polymerizable monomer (A-2).

8. The dental curable composition according to claim 7, wherein the polymerizable monomer (A-2) is a (meth)acrylic polymerizable monomer.

9. The dental curable composition according to any one of claims 1 to 8, wherein the polymerization initiator (D) comprises a photopolymerization initiator (D-1).

10. The dental curable composition according to any one of claims 1 to 9, which further comprises a filler (E).

11. The dental curable composition according to claim 10, wherein the filler (E) comprises a hydrophobic silica (E-1).

12. The dental curable composition according to claim 11, wherein the hydrophobic silica (E-1) has an average primary particle diameter of 15 to 100 nm.

13. The dental curable composition according to any one of claims 10 to 12, wherein the content of the filler (E) is 0.1 to 20 mass%.

14. The dental curable composition according to any one of claims 1 to 13, which further comprises a polysiloxane (F).

15. The dental curable composition according to claim 14, wherein the polysiloxane (F) is liquid at 25°C.

16. The dental curable composition according to claim 14 or 15, wherein the polysiloxane (F) comprises a polysilsesquioxane (F-1).

**17.** The dental curable composition according to any one of claims 1 to 16, wherein the dental curable composition is a coating agent for coating a base material.

**18.** A dental prosthesis comprising a resin layer and a base material, the resin layer being a layer cured through polymerization of a dental curable composition of any one of claims 1 to 17.

**19.** The dental prosthesis according to claim 18, wherein the base material comprises at least one selected from the group consisting of zirconia, alumina, fused quartz, lithium silicate glass, an acrylic resin, and a composite material containing a polymer resin and an inorganic filler.

**20.** The dental prosthesis according to claim 19, wherein the zirconia comprises zirconia, and a stabilizing agent capable of preventing a phase transformation of zirconia.

**21.** The dental prosthesis according to any one of claims 18 to 20, wherein the resin layer has a thickness of 1 μm to 500 μm.

## INTERNATIONAL SEARCH REPORT

International application No.
**PCT/JP2022/047911**

**A. CLASSIFICATION OF SUBJECT MATTER**

***A61K 6/20***(2020.01)i; ***A61K 6/17***(2020.01)i; ***A61K 6/60***(2020.01)i; ***A61K 6/62***(2020.01)i; ***A61K 6/65***(2020.01)i; ***A61K 6/76***(2020.01)i; ***A61K 6/78***(2020.01)i; ***A61K 6/802***(2020.01)i; ***A61K 6/818***(2020.01)i
FI: A61K6/20; A61K6/60; A61K6/65; A61K6/78; A61K6/62; A61K6/76; A61K6/802; A61K6/818; A61K6/17

According to International Patent Classification (IPC) or to both national classification and IPC

**B. FIELDS SEARCHED**

Minimum documentation searched (classification system followed by classification symbols)

A61K6/20; A61K6/17; A61K6/60; A61K6/62; A61K6/65; A61K6/76; A61K6/78; A61K6/802; A61K6/818

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2020-509060 A (DENTSPLY SIRONA INC.) 26 March 2020 (2020-03-26) claims, tables 1-3, paragraphs [0001], [0018]-[0021], [0027] | 1-21 |
| Y | WO 2020/106349 A1 (DENTSPLY SIRONA INC.) 28 May 2020 (2020-05-28) claims 1-3, 6-7, 13, page 1, "FIELD OF THE DISCLOSURE", pages 14-31, "EXPERIMENTAL PROCEDURES" | 1-21 |
| Y | WO 2021/125246 A1 (KURARAY NORITAKE DENTAL INC.) 24 June 2021 (2021-06-24) claims, paragraphs [0002], [0143]-[0145], examples 1-7 | 1-21 |
| Y | JP 2014-15408 A (TOKUYAMA DENTAL CORP.) 30 January 2014 (2014-01-30) claims, paragraph [0008], examples 1-13 | 1-21 |
| Y | JP 2010-222466 A (NORITAKE CO., LIMITED) 07 October 2010 (2010-10-07) claims, paragraphs [0018]-[0025], [0064]-[0072] | 1-21 |
| Y | JP 2019-116471 A (TOKUYAMA DENTAL CORP) 18 July 2019 (2019-07-18) claims, paragraphs [0008], [0045]-[0050], [0076]-[0079], examples | 1-21 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 March 2023** | **04 April 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

INTERNATIONAL SEARCH REPORT

International application No. **PCT/JP2022/047911**

C. DOCUMENTS CONSIDERED TO BE RELEVANT

| Category* | Citation of document, with indication, where appropriate, of the relevant passages | Relevant to claim No. |
|---|---|---|
| Y | US 2011/0144230 A1 (KOLTISKO, Bernard) 16 June 2011 (2011-06-16) claims, paragraphs [0004], [0033]-[0038], tables I-IV | 1-21 |
| Y | WO 2011/074222 A1 (KURARAY MEDICAL INC.) 23 June 2011 (2011-06-23) claims, paragraph [0012], production examples 6-21, examples | 1-21 |
| Y | JP 2016-153382 A (KURARAY NORITAKE DENTAL INC.) 25 August 2016 (2016-08-25) claims, paragraph [0018], production examples 2-9, table 1 | 1-21 |
| Y | JP 2018-89312 A (KURARAY NORITAKE DENTAL INC.) 14 June 2018 (2018-06-14) claims, paragraph [0008], examples | 1-21 |
| P, X | JP 7090366 B1 (YAMAKIN CO., LTD.) 24 June 2022 (2022-06-24) claims, paragraphs [0053], [0066], examples | 1-3, 9-10, 17, 21 |
| A | JP 2021-524395 A (3M INNOVATIVE PROPERTIES COMPANY) 13 September 2021 (2021-09-13) entire text | 1-21 |

Form PCT/ISA/210 (second sheet) (January 2015)

**INTERNATIONAL SEARCH REPORT**
**Information on patent family members**

International application No.
**PCT/JP2022/047911**

| Patent document cited in search report | Publication date (day/month/year) | Patent family member(s) | Publication date (day/month/year) |
|---|---|---|---|
| JP 2020-509060 A | 26 March 2020 | US 2018/0250201 A1<br>claims, tables 1-3, paragraphs [0001], [0058]-[0060], [0063]<br>EP 3589262 A1<br>CN 110430858 A | |
| WO 2020/106349 A1 | 28 May 2020 | EP 3883976 A1<br>CN 113099718 A<br>JP 2022-509108 A | |
| WO 2021/125246 A1 | 24 June 2021 | EP 4079280 A1<br>claims, paragraphs [0002], [0145]-[0147], examples | |
| JP 2014-15408 A | 30 January 2014 | (Family: none) | |
| JP 2010-222466 A | 07 October 2010 | US 2012/0012789 A1<br>claims, paragraphs [0020]-[0027], [0068]-[0075], table 2<br>EP 2412691 A1<br>KR 10-2011-0138393 A<br>CN 102438964 A | |
| JP 2019-116471 A | 18 July 2019 | US 2020/0330333 A1<br>claims, paragraphs [0012], [0100]-[0104], [0136]-[0147], examples<br>EP 3733150 A1<br>CN 111491600 A | |
| US 2011/0144230 A1 | 16 June 2011 | EP 2521526 A2 | |
| WO 2011/074222 A1 | 23 June 2011 | US 2011/0257292 A1<br>claims, paragraph [0014], preparation examples 6-21, examples<br>EP 2380551 A1<br>CN 102341088 A | |
| JP 2016-153382 A | 25 August 2016 | (Family: none) | |
| JP 2018-89312 A | 14 June 2018 | (Family: none) | |
| JP 7090366 B1 | 24 June 2022 | (Family: none) | |
| JP 2021-524395 A | 13 September 2021 | US 2021/0214504 A1<br>entire text<br>EP 3797128 A1<br>CN 112154171 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018089312 A
- JP 2001149385 A
- JP 2005154312 A
- JP 2021054813 A
- JP 2016153382 A
- WO 2019026809 A
- JP 2020509060 T
- JP 2019116471 A
- JP H0357916 B